# EUROPEAN PATENT APPLICATION

(11) **EP 4 817 543 A1**
(43) Date of publication of application: **07.10.2026**
(21) Application number: 26168784.2
(22) Date of filing: 27.03.2026
(51) Int. Cl.: A61B 18/14, A61B 18/00

(54) **LARGE AREA FOCAL PULSED FIELD ABLATION CATHETER**

(30) Priority: 31.03.2025 US 202563781293 P; 17.06.2025 EP 25183221
(71) Applicant: CRC EP, Inc., Lake Oswego, OR 97035 (US)
(72) Inventor: Töpfer, Fritzi, 12046 Berlin (DE); Holzinger, Steffen, 12437 Berlin (DE); Panescu, Dorin, San Jose, 95136 (US); Nguyen, Tho, Huntington Beach, 92646 (US); Hata, Cary, Irvine, 92620 (US)
(74) Representative: Biotronik Corporate Services SE

(57) **Abstract**

An ablation catheter for treatment of patient tissue by delivery of high-voltage pulses is provided. The ablation catheter has an ablation portion forming at least one, optionally two loop sections in a round structure or a three-dimensional spiral. The ablation portion has a plurality of ablation electrodes positioned along a first loop section comprising the innermost or distal most portion in the form of a round structure or of the three-dimensional spiral. The ablation electrodes have first edges facing the interior of the round structure or the three-dimensional spiral and the distance between any two first edges is not greater than 12mm. In operation, each ablation electrode produces a respective ablation region and these ablation regions may overlap to form a single contiguous ablation region.

## Description

The present application is generally related to treatment of tissue using pulsed field ablation (PFA) and more particularly related to generating a single contiguous ablation region in a single PFA shot.

Pulsed field ablation is a promising method for ablation of cardiac tissue and the treatment of arrhythmia. Conventional single shot PFA catheters allow for relatively precise ablation of specific points on cardiac tissue. However, single shot PFA catheters have limited use cases such as ablation of pulmonary veins and posterior walls or cavotricuspid isthmus. It is also difficult to reliably align the single shot PFA catheter to effectively ablate large regions of tissue.

Hence, there is a need to improve the methods and the catheters for pulsed field ablation and in particular pulsed field ablation of cardiac tissue and the treatment of arrhythmia.

Therefore, it is the object of the present invention to provide an improved catheter system and a method for treating cardiac tissue by pulsed field ablation.

The above object is solved by the subject-matter of the claims as defined herein.

In some aspects, the techniques described herein relate to an ablation catheter for treatment of patient tissue by delivery of high-voltage pulses including: a catheter shaft having an ablation portion at a distal end of the catheter shaft, wherein the ablation portion includes at least one, optionally two loop sections forming a round structure or a three-dimensional spiral. If a spiral the at least two loop sections are configured to transition between an uncompressed position where the ablation portion is not restricted by any external force and a compressed position where the ablation portion is compressed by an external force. A plurality of electrodes is positioned along the ablation portion, the plurality of electrodes including a plurality of ablation electrodes. A first loop section includes the innermost or distal most portion in form of the round structure or of the three-dimensional spiral. In the three-dimensional spiral a second loop section, adjacent to the first loop section is arranged, wherein the second loop section does not include any ablation electrodes. The catheter may include a connection member between the shaft and the first loop section. Also, a first ablation electrode is positioned on the first loop section, the first ablation electrode having a first edge facing an interior of the round structure or the three dimensional spiral in a plan view; a second ablation electrode is positioned on the first loop section, the second ablation electrode having a first edge facing an interior of the round structure or the three dimensional spiral in a plan view; a third ablation electrode is positioned on the first loop section, the third ablation electrode having a first edge facing an interior of the round structure or the three dimensional spiral in a plan view; a fourth ablation electrode is positioned on the first loop section, the fourth ablation electrode having a first edge facing an interior of the round structure or the three dimensional spiral in a plan view; wherein a distance across the interior of the round structure or the three dimensional spiral in a plan view between the first edge of any two of the first ablation electrode, the second ablation electrode, the third ablation electrode, and the fourth ablation electrode, is not greater than 12mm in the pressed down or compressed position, and optionally wherein the first edge of the first ablation electrode and the first edge of the third ablation electrode substantially face each other in the pressed down position for the round structure or compressed position for the spiral, wherein the first edge of the second ablation electrode and the first edge of the fourth ablation electrode substantially face each other in the pressed down position for the round structure or compressed position for the spiral, and preferably wherein the first edge of the first ablation electrode and the first edge of the third ablation electrode are substantially perpendicular to the first edge of the second ablation electrode and the first edge of the fourth ablation electrode in the pressed down or compressed position.

In light of the present disclosure "round structure" shall mean any loop structure closed or open that has a round shape and encloses at least partially an interior area. Shapes that fall within the term "round structure" can be circular, oval, elliptic or curved enclosing more than 180 degrees. Further, "round structure" may be a loop structure enclosing in interior by more than 270 degrees, preferably by more than 300 degrees, more preferably by more than 330 degrees, and most preferably more than 355 degrees in a plan view.

Also for all embodiments as described herein above and below, the plurality of ablation electrodes on the first loop section may be evenly distributed about the loop section. For example, the distance between each electrode of the plurality of ablation electrodes on the first loop section may not exceed 360 degrees divided by the number of electrodes ±15 degrees, preferably ±10, more preferably ±5 degrees. Preferably, the distance between each electrode may not exceed 95 degrees, in particular, when the first loop comprises four ablation electrodes. Preferably, the distance between each electrode may not exceed 65 degrees, in particular, when the first loop comprises six ablation electrodes.

In one embodiment the ablation catheter comprises only one loop section, preferably in form of a round structure, more preferably in the form of a curved line or a circular structure, most preferably in a circular structure preferably being circular, oval, elliptic or curved enclosing more than 180 degrees.

In that the techniques described herein specifically also relate to an ablation catheter for treatment of patient tissue by delivery of high-voltage pulses comprising:
a catheter shaft having an ablation portion at a distal end of the catheter shaft, wherein the ablation portion comprises one loop section forming a round structure;
a plurality of electrodes positioned along the ablation portion, the plurality of electrodes comprising a plurality of ablation electrodes;
the first loop section comprising the distal most portion in the form of a round structure;
   a first ablation electrode positioned on the first loop section, the first ablation electrode having a first edge facing an interior of the round structure in a plan view;
a second ablation electrode positioned on the first loop section, the second ablation electrode having a first edge facing an interior of the round structure in a plan view;
a third ablation electrode positioned on the first loop section, the third ablation electrode having a first edge facing an interior of the round structure in a plan view;
a fourth ablation electrode positioned on the first loop section, the fourth ablation electrode having a first edge facing an interior of the round structure in a plan view;
an optional fifth ablation electrode positioned on the first loop section, the optional fifth ablation electrode having a first edge facing an interior of the round structure in a plan view;
an optional sixth ablation electrode positioned on the first loop section, the optional sixth ablation electrode having a first edge facing an interior of the round structure in a plan view;
wherein a distance across the interior of the round structure in a plan view between the first edge of any two of the first ablation electrode, the second ablation electrode, the third ablation electrode, the fourth ablation electrode, the optional fifth ablation electrode, and the optional sixth ablation electrode, is not greater than 12mm in a pressed down position, and optionally
wherein the first ablation electrode, the second ablation electrode, the third ablation electrode, the fourth ablation electrode, the optional fifth ablation electrode, and the optional sixth ablation electrode, are evenly distributed relative to each other in the pressed down position, such that each of the first ablation electrode, the second ablation electrode, the third ablation electrode, the fourth ablation electrode, the optional fifth ablation electrode, and the optional sixth ablation electrode occupies a corner or a side, in particular the center area of a side, of a square or a hexagon in a plan view.

In one embodiment, the loop section has four electrodes and the first edge of the first ablation electrode and the first edge of the third ablation electrode are substantially perpendicular to the first edge of the second ablation electrode and the first edge of the fourth ablation electrode in the pressed down or compressed position.

Further, the techniques described herein relate to an ablation catheter for treatment of patient tissue by delivery of high-voltage pulses comprising:
a catheter shaft having an ablation portion at a distal end of the catheter shaft, wherein the ablation portion comprises one loop section forming a round structure;
a plurality of electrodes positioned along the ablation portion, the plurality of electrodes comprising a plurality of ablation electrodes;
the first loop section comprising the distal most portion in the form of a round structure;
a first ablation electrode positioned on the first loop section, the first ablation electrode having a first edge facing an interior of the round structure in a plan view;
a second ablation electrode positioned on the first loop section, the second ablation electrode having a first edge facing an interior of the round structure in a plan view;
a third ablation electrode positioned on the first loop section, the third ablation electrode having a first edge facing an interior of the round structure in a plan view;
a fourth ablation electrode positioned on the first loop section, the fourth ablation electrode having a first edge facing an interior of the round structure in a plan view;
wherein a distance across the interior of the round structure in a plan view between the first edge of any two of the first ablation electrode, the second ablation electrode, the third ablation electrode, and the fourth ablation electrode, is not greater than 12mm in a pressed down position, and optionally
wherein the first ablation electrode, the second ablation electrode, the third ablation electrode, the fourth ablation electrode, are evenly distributed relative to each other in the pressed down position, such that each of the first ablation electrode, the second ablation electrode, the third ablation electrode, the fourth ablation electrode, occupies a corner or a side, in particular the center area of a side, of a square in a plan view and/or wherein the first edge of the first ablation electrode and the first edge of the third ablation electrode are substantially perpendicular to the first edge of the second ablation electrode and the first edge of the fourth ablation electrode in the pressed down or compressed position. The ablation portion may comprise only one loop section.

Moreover, the technique described herein relate to an ablation catheter for treatment of patient tissue by delivery of high-voltage pulses comprising: a catheter shaft having an ablation portion at a distal end of the catheter shaft, wherein the ablation portion comprises one loop section; a plurality of electrodes positioned along the ablation portion, the plurality of electrodes comprising a plurality of ablation electrodes; the loop section comprising the distal most portion in the form of a round structure; a first ablation electrode positioned on the loop section; a second ablation electrode positioned on the loop section; a third ablation electrode positioned on the loop section; a fourth ablation electrode positioned on the loop section; an optional fifth ablation electrode positioned on the loop section; an optional sixth ablation electrode positioned on the loop section, wherein a distance across the interior of the round structure in a plan view between interior aspects, in particular edges of any two electrodes, is not greater than 12mm in a deployed position.

In one embodiment the ablation catheter has four electrodes, and the round structure is in the form of a circle structure being circular, oval, elliptic or curved enclosing more than 180 degrees. Further, the first ablation electrode, the second ablation electrode, the third ablation electrode, the fourth ablation electrode, may be evenly distributed relative to each other in the pressed down or plan position, such that each of the first ablation electrode, the second ablation electrode, the third ablation electrode, the fourth ablation electrode, occupies a corner or a side, in particular the center area of a side, of a square in a plan view or wherein the first edge of the first ablation electrode and the first edge of the third ablation electrode are substantially perpendicular to the first edge of the second ablation electrode and the first edge of the fourth ablation electrode in the pressed down or plan position.

In another embodiment the ablation catheter has six electrodes, and the round structure is in the form of a circle structure being circular, oval, elliptic or curved enclosing more than 180 degrees. Further, the first ablation electrode, the second ablation electrode, the third ablation electrode, the fourth ablation electrode, the fifth ablation electrode, and the sixth ablation electrode, are evenly distributed relative to each other in the pressed down or plan position, such that each of the first ablation electrode, the second ablation electrode, the third ablation electrode, the fourth ablation electrode, the fifth ablation electrode, and the sixth ablation electrode occupies a corner or a side, in particular the center area of a side, of a hexagon in a plan view.

In some aspects, the techniques described herein relate to an ablation catheter for treatment of patient tissue by delivery of high-voltage pulses including: a catheter shaft having an ablation portion at a distal end of the catheter shaft, wherein the ablation portion includes at least one, optionally two loop sections forming a round structure or a three-dimensional spiral. If a spiral, the at least two loop sections are configured to transition between an uncompressed position where the ablation portion is not restricted by any external force and a compressed position where the ablation portion is compressed by an external force. A plurality of electrodes is positioned along the ablation portion, the plurality of electrodes including a plurality of ablation electrodes. A first loop section includes the innermost or distal most portion in the form of the round structure or of the three-dimensional spiral. In the three-dimensional spiral a second loop section, adjacent to the first loop section is arranged, wherein the second loop section does not include any ablation electrodes. The catheter may include a connection member between the shaft and the first loop section. Also, a first ablation electrode is positioned on the first loop section, the first ablation electrode having a first edge facing an interior of the round structure or the three dimensional spiral in a plan view; a second ablation electrode is positioned on the first loop section, the second ablation electrode having a first edge facing an interior of the round structure or the three dimensional spiral in a plan view; a third ablation electrode is positioned on the first loop section, the third ablation electrode having a first edge facing an interior of the round structure or the three dimensional spiral in a plan view; a fourth ablation electrode is positioned on the first loop section, the fourth ablation electrode having a first edge facing an interior of the round structure or the three dimensional spiral in a plan view; a fifth ablation electrode is positioned on the first loop section, the fifth ablation electrode having a first edge facing an interior of the round structure or the three dimensional spiral in a plan view; a sixth ablation electrode is positioned on the first loop section, the sixth ablation electrode having a first edge facing an interior of the round structure or the three dimensional spiral in a plan view; wherein a distance across the interior of the round structure or the three dimensional spiral in a plan view between the first edge of any two of the first ablation electrode, the second ablation electrode, the third ablation electrode, the fourth ablation electrode, the fifth ablation electrode, and the sixth ablation electrode, is not greater than 12mm in the pressed down position for the round structure or compressed position, and optionally wherein the first ablation electrode, the second ablation electrode, the third ablation electrode, the fourth ablation electrode, the fifth ablation electrode, and the sixth ablation electrode, are evenly distributed relative to each other in the pressed down or compressed position, such that each of the first ablation electrode, the second ablation electrode, the third ablation electrode, the fourth ablation electrode, the fifth ablation electrode, and the sixth ablation electrode occupies a corner or a side, in particular the center area of a side, of a hexagon in a plan view.

In some aspects, the techniques described herein relate to an ablation catheter for treatment of patient tissue by delivery of high-voltage pulses including: a catheter shaft having an ablation portion at a distal end of the catheter shaft, wherein the ablation portion includes at least one, optionally two loop sections forming a round structure or a three-dimensional spiral. If a spiral, the at least two loop sections are configured to transition between an uncompressed position where the ablation portion is not restricted by any external force and a compressed position where the ablation portion is compressed by an external force. A plurality of electrodes is positioned along the ablation portion, the plurality of electrodes including a plurality of ablation electrodes. A first loop section includes the innermost or distal most portion in the form of the round structure or of the three-dimensional spiral. In the three- dimensional spiral a second loop section, adjacent to the first loop section is arranged, wherein the second loop section does not include any ablation electrodes. The catheter may include a connection member between the shaft and the first loop section. Also, a first ablation electrode is positioned on the first loop section, the first ablation electrode having a first edge facing an interior of the round structure or the three dimensional spiral in a plan view; a second ablation electrode is positioned on the first loop section, the second ablation electrode having a first edge facing an interior of the round structure or the three dimensional spiral in a plan view; wherein a distance across the interior of the round structure or the three dimensional spiral in a plan view between the first edge of the first ablation electrode and the first edge of the second ablation electrode, is not greater than 12mm in the pressed down position for the round structure compressed position, and optionally wherein the first ablation electrode produces a first ablation region and the second ablation electrode produces a second ablation region, and the first ablation region and the second ablation region overlap to form a single contiguous ablation region.

In some aspects, the techniques described herein relate to a method for treatment of patient tissue by delivery of high-voltage pulses including: providing an ablation catheter having an ablation portion including a plurality of ablation electrodes, the ablation portion including at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes. When the catheter has at least two loop sections, they are configured to transition between an uncompressed position where the ablation portion is not restricted by any external force and a compressed position where the ablation portion is compressed by an external force. In both embodiments such as round structure or three-dimensional spiral the catheter has a first loop section, wherein the first loop section includes the innermost portion of the round structure or the three-dimensional spiral and further includes a plurality of ablation electrodes, each of the plurality of ablation electrodes including a first edge facing an interior of the round structure or the three dimensional spiral in a plan view, wherein a distance across the interior of the round structure or the three dimensional spiral in a plan view between the first edge of any two of the ablation electrodes is not greater than 12mm in a pressed down or the compressed position; and energizing the plurality of ablation electrodes with high voltage charge-balanced pulsed electric fields which are delivered in a monopolar arrangement to form a single contiguous ablation region.

In one embodiment the ablation catheter comprises only one loop section, preferably in form of a round structure, more preferably in the form of a curved line or a circular structure, most preferably in a circular structure preferably being circular, oval, elliptic or curved enclosing more than 180 degrees.

In some aspects, the techniques described herein relate to a method for treatment of patient tissue by delivery of high-voltage pulses including: providing an ablation catheter having an ablation portion including a plurality of ablation electrodes, the ablation portion including at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes. When the catheter has at least two loop sections, they are configured to transition between an uncompressed position where the ablation portion is not restricted by any external force and a compressed position where the ablation portion is compressed by an external force. In both embodiments such as round structure or three-dimensional spiral the catheter has a first loop section, wherein the first loop section includes the innermost portion of the round structure or the three-dimensional spiral includes a plurality of ablation electrodes, each of the plurality of ablation electrodes including a first edge facing an interior of the round structure or the three dimensional spiral in a plan view, wherein a distance across the interior of the round structure or the three dimensional spiral in a plan view between the first edge of any two of the ablation electrodes is not greater than 12mm in a pressed down position or the compressed position; and energizing the plurality of ablation electrodes with high voltage charge-balanced pulsed electric fields which are delivered in a monopolar arrangement to form a single contiguous ablation region. In one embodiment the ablation catheter comprises only one loop section, preferably in form of a round structure, more preferably in the form of a curved line or a circular structure, most preferably in a circular structure preferably being circular, oval, elliptic or curved enclosing more than 180 degrees.

In some aspects, the techniques described herein relate to an ablation catheter for treatment of patient tissue by delivery of high-voltage pulses including: a catheter shaft having an ablation portion at a distal end of the catheter shaft, wherein the ablation portion includes at least one, optionally two loop sections forming a round structure or a three-dimensional spiral. In the embodiment of a three-dimensional spiral, the at least two loop sections are configured to transition between an uncompressed position where the ablation portion is not restricted by any external force and a compressed position where the ablation portion is compressed by an external force. In both embodiments such as round structure or three-dimensional spiral the plurality of ablation electrodes are arranged along a first loop section including the innermost portion of the round structure or the three-dimensional spiral; a second loop section, adjacent to the first loop section, wherein the second loop section does not include any ablation electrodes; wherein the plurality of ablation electrodes are arranged at vertices or a side, in particular the center area of a side, of a rectangular or hexagonal polygon in a plan view relative to each other in a pressed down position or the compressed position, and wherein the distance between any two ablation electrodes does not exceed 12 mm in the pressed down or the compressed position.

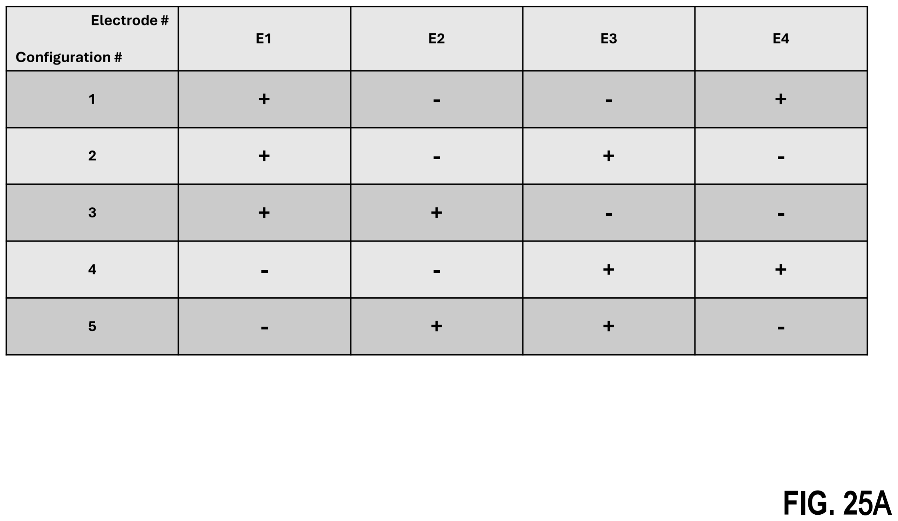

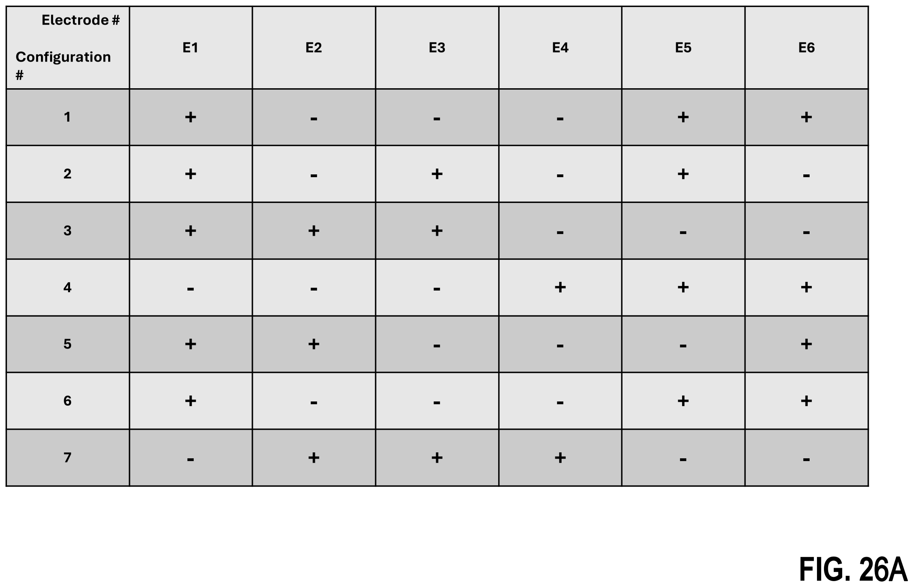

Another aspect of the present invention is related to a method of treatment of patient tissue by delivery of high-voltage pulses by one of the catheters as described herein above and below. The method of treatment is directed to electrode configurations exemplary according to FIGs 25A and 26 A. Electrode configurations relate to the specific distribution of the polarities to the ablation electrodes. Hence, the electrode configurations define which electrode of the plurality of electrode have a "+" polarity and which electrode have a "-" polarity, and in specific examples which electrode stay silent during a pulse train. The ablation portion comprising a plurality of ablation electrodes, in particular on the first loop section may comprise at described above two, four or six electrodes. It is comprised by the present invention that the first loop section may comprise three, five, seven, eight or more electrodes. Also for these embodiments, the distance between any two ablation electrodes does not exceed 12 mm in the pressed down or the compressed position. For the electrode configurations it is preferred that the same number of electrodes have a "+" polarity and the other electrodes having a "-" polarity. By operating the different electrodes with different polarities, the current flow of the pulses from the ablation portion can be controlled. In case of an uneven number of electrodes the residual one can have a "+" polarity or a "-" polarity or can remain silent. The method of treatment of patient tissue by delivery of high-voltage pulses by one of the catheters as described herein may also comprise combinations of configurations of the plurality of electrodes. This means that during a first pulse train a first configuration is applied, while for a following second pulse train the same or a different configuration is applied. A combination of configurations may comprise at least two pulse trains, and may have three, four, five, six or more configurations, being the same configuration or different ones. It may also be that one configuration is repeated directly have the first application or later, after a different configuration has been applied. Taking the simplest example with two ablation electrodes, in a first pulse train electrode 1 (E1) has a "+" polarity, while electrode 2 (E2) has a "-" polarity, for the second pulse train E1 may then have a "-" polarity and E2 may have a "+" polarity, or the same polarity for both electrodes is again applied. For a potential following third pulse train the polarity may change or may stay the same. It is preferred that at least once for the treatment as described herein the configuration for the electrode is changed to one other possible configuration, and thereby the current flow is changed. It has been observed that changing the current flow at least once, thorough tissue in area and depth can be achieved in very short ablation times, without the need for repeated long ablation sessions. For embodiments of the catheter as described herein with more than two electrodes it is preferred that the configuration is changed twice, in particular that three different configurations are applied and thereby no configuration has been repeatedly applied.

In some aspects, the techniques described herein relate to a method for treatment of patient tissue by delivery of high-voltage pulses including:
providing an ablation catheter having an ablation portion including a plurality of ablation electrodes, the ablation portion including at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes. When the catheter has at least two loop sections, they are configured to transition between an uncompressed position where the ablation portion is not restricted by any external force and a compressed position where the ablation portion is compressed by an external force. In both embodiments such as round structure or three-dimensional spiral the catheter has a first loop section, wherein the first loop section includes the innermost portion in the form of the round structure or the three-dimensional spiral and further includes a plurality of ablation electrodes, each of the plurality of ablation electrodes including a first edge facing an interior of the round structure or the three dimensional spiral in a plan view, wherein a distance across the interior of the round structure or the three dimensional spiral in a plan view between the first edge of any two of the ablation electrodes is not greater than 12mm in a pressed down position or the compressed position; and energizing the plurality of ablation electrodes with high voltage charge-balanced pulsed electric fields, preferably in a monopolar arrangement to form a single contiguous ablation region,
wherein the electrodes of the plurality of ablation electrodes have a first configuration, wherein the same number of electrodes have a "+" polarity and a "-" polarity, or the number of electrodes with one polarity differs from the number of electrodes with the other polarity by only one (1).

Also, in some aspects the techniques described herein relate to a method for treatment of patient tissue by delivery of high-voltage pulses including:
providing an ablation catheter having an ablation portion including a plurality of ablation electrodes, the ablation portion including one loop section forming a round structure at a distal end and a proximal end adapted to connect to an electronic control unit configured to provide monopolar, bipolar, or a combination of monopolar and bipolar energy a plurality of ablation electrodes, each of the plurality of ablation electrodes including a first edge facing an interior of the round structure in a plan view, wherein a distance across the interior of the round structure in a plan view between the first edge of any two of the ablation electrodes is not greater than 12mm in a pressed down position; and energizing the plurality of ablation electrodes with high voltage charge-balanced pulsed electric fields, preferably in a monopolar arrangement to form a single contiguous ablation region, preferably
wherein the electrodes of the plurality of ablation electrodes have a first configuration, wherein the same number of electrodes have a "+" polarity and a "-" polarity, or the number of electrodes with one polarity differs from the number of electrodes with the other polarity by only one (1).

In one embodiment the ablation catheter comprises only one loop section, preferably in form of a round structure, more preferably in the form of a curved line or a circular structure, most preferably in a circular structure preferably being circular, oval, elliptic or curved enclosing more than 180 degrees.

In one, such as the first, electrode configuration specific electrodes have a "+" polarity and other electrodes have a "-" polarity. In a different, such as a second, electrode configuration at least one electrode pair has a different polarity than in the first or any other configuration.

Also, in some aspects the techniques described herein relate to a method for treatment of patient tissue by delivery of high-voltage pulses including:
providing an ablation catheter for treatment of patient tissue by delivery of high-voltage pulses comprising: a catheter shaft having an ablation portion at a distal end of the catheter shaft and a proximal end adapted to connect to an electronic control unit configured to provide monopolar, bipolar, or a combination of monopolar and bipolar energy a plurality of ablation electrodes, wherein the ablation portion comprises one loop section; a plurality of electrodes positioned along the ablation portion, the plurality of electrodes comprising a plurality of ablation electrodes; the loop section comprising the distal most portion in the form of a round structure; a first ablation electrode positioned on the loop section; a second ablation electrode positioned on the loop section; a third ablation electrode positioned on the loop section; a fourth ablation electrode positioned on the loop section; an optional fifth ablation electrode positioned on the loop section; an optional sixth ablation electrode positioned on the loop section, wherein a distance across the interior of the round structure in a plan view between interior aspects, in particular edges of any two electrodes, is not greater than 12mm in a deployed position, and energizing the plurality of ablation electrodes with high voltage charge-balanced pulsed electric fields, preferably in a monopolar arrangement to form a single contiguous ablation region,
wherein the electrodes of the plurality of ablation electrodes have a first configuration, wherein the same number of electrodes have a "+" polarity and a "-" polarity, or the number of electrodes with one polarity differs from the number of electrodes with the other polarity by only one (1).

The method above may in particular be conducted by applying the electrode configurations according to FIGs 25A and 26 A; and further combinations of the electrode configurations according to FIGs 25A and 26 A.

In one embodiment of the above method the ablation catheter has four electrodes, and the round structure is in the form of a circular structure or loop, preferably being circular, oval, elliptic or curved enclosing more than 180 degrees. Further, the first ablation electrode, the second ablation electrode, the third ablation electrode, the fourth ablation electrode, may be evenly distributed relative to each other in the pressed down or plan position, such that each of the first ablation electrode, the second ablation electrode, the third ablation electrode, the fourth ablation electrode, occupies a corner or a side, in particular the center area of a side, of a square in a plan view or wherein the first edge of the first ablation electrode and the first edge of the third ablation electrode are substantially perpendicular to the first edge of the second ablation electrode and the first edge of the fourth ablation electrode in the pressed down or plan position.

In another embodiment of the above method the ablation catheter has six electrodes, and the round structure is in the form of a circular structure or loop, preferably being circular, oval, elliptic or curved enclosing more than 180 degrees. Further, the first ablation electrode, the second ablation electrode, the third ablation electrode, the fourth ablation electrode, the fifth ablation electrode, and the sixth ablation electrode, are evenly distributed relative to each other in the pressed down or plan position, such that each of the first ablation electrode, the second ablation electrode, the third ablation electrode, the fourth ablation electrode, the fifth ablation electrode, and the sixth ablation electrode occupies a corner or a side, in particular the center area of a side, of a hexagon in a plan view.

In one embodiment of the methods as described above, the step of energizing the plurality of ablation electrodes with high voltage charge-balanced pulsed electric fields is conducted by at least two pulse trains, preferably wherein the at least two pulse trains are provided by a combination of at least two different configurations. In another embodiment, the method is conducted by a combination of at least three different configurations. The step of energizing the plurality of ablation electrodes with high voltage charge-balanced pulsed electric fields may also be conducted by using different configurations, wherein specific electrode configurations are used repeatedly.

Other features and advantages of the present invention will become more readily apparent to those of ordinary skill in the art after reviewing the following detailed description and accompanying drawings.

The various features and advantages of the present invention may be more readily understood with reference to the following detailed description and the embodiments shown in the drawings. Herein schematically and exemplarily,
- FIG. 1: illustrates an example distal end of an example ablation catheter in a perspective side view;
- FIG. 2: illustrates an example distal end of the example ablation catheter of FIG. 1 with electrode numbering in a top view;
- FIG. 3: illustrates an example delivery path for an example ablation catheter leading to a pulmonary vein ostium of a human heart;
- FIG. 4: illustrates an example part of the electric control of the electrode leads for the example ablation catheter of FIG. 1;
- FIG. 5: illustrates an example part of the electric control of the electrode leads for the example ablation catheter of FIG. 1;
- FIG. 6A: illustrates a side view of an example ablation portion of an ablation catheter arranged in a corkscrew embodiment;
- FIG. 6B: illustrates a plan view of an example ablation portion of an ablation catheter arranged in a corkscrew embodiment;
- FIG. 7A: illustrates a side view of an example ablation portion of an ablation catheter arranged in a plunger embodiment;
- FIG. 7B: illustrates a plan view of an example ablation portion of an ablation catheter arranged in a plunger embodiment;
- FIGS. 8A,: 8B, and 8C illustrate plan view examples of alternative arrangements of ablation electrodes on a compressed ablation portion of an ablation;
- FIG. 9: illustrates an example lesion depth for a 1st example of an ablation catheter at various voltages;
- FIG. 10: illustrates an example lesion width for the 1st example of an ablation catheter of at various voltages;
- FIG. 11: illustrates an example lesion depth for a 2^{nd} example of an ablation catheter at various voltages;
- FIG. 12: illustrates an example lesion width for the 2^{nd} example of an ablation catheter at various voltages;
- FIG. 13: illustrates an example lesion depth for a 3^{rd} example of an ablation catheter at various voltages;
- FIG. 14: illustrates an example lesion width for the 3^{rd} example of an ablation catheter at various voltages;
- FIG. 15: illustrates an example lesion depth for a 4^{th} example of an ablation catheter at various voltages;
- FIG. 16: illustrates an example lesion width for the 4rd example of an ablation catheter at various voltages;
- FIG. 17: illustrates an example lesion depth for a 5^{th} example of an ablation catheter at various voltages;
- FIG. 18: illustrates an example lesion width at 2mm depth for the 5^{th} example of an ablation catheter at various voltages;
- FIG. 19: illustrates an example lesion width at 0.5mm depth for the 5^{th} example of an ablation catheter at various voltages;
- FIG. 20: is a flow diagram illustrating an example process for treatment of patient tissue by delivery of high-voltage pulses;
- FIG. 21 A: shows an exemplary catheter arrangement with four electrodes according to the present invention;
- FIGs 21 B-C: show an example lesion on a potato in width and depth by using the catheter arrangement of FIG. 21 A;
- FIGs 22 A-C: show another series of example lesions in width, breadth and detailed depth using the catheter arrangement of FIG. 21 A using the two electrode configurations of FIGs 25A and B consecutively;
- FIG. 23 A: shows a comparative catheter arrangement of the inner four electrodes;
- FIG.s 23 B-C: show an example lesion on a potato in width and depth by using the catheter arrangement of FIG. 23 A;
- FIG. 24: shows an exemplary catheter arrangement with six electrodes according to the present invention;
- FIGs 25 A-C: show polarity electrode configurations for the exemplary catheter arrangement with four electrodes. In FIGs 25 B and C the current flow is indicated by arrows; and
- FIGs 26 A-C: show polarity electrode configurations for the exemplary catheter arrangement with six electrodes. In FIGs 26 B and C the current flow is indicated by arrows.

Disclosed herein are systems and methods for single shot pulsed field ablation that generates a large contiguous ablation area on the target tissue.

After reading this description it will become apparent to one skilled in the art how to implement the invention in various alternative embodiments and alternative applications. However, although various embodiments of the present invention will be described herein, it is understood that these embodiments are presented by way of example only, and not limitation. As such, this detailed description of various alternative embodiments should not be construed to limit the scope or breadth of the present invention as set forth in the appended claims.

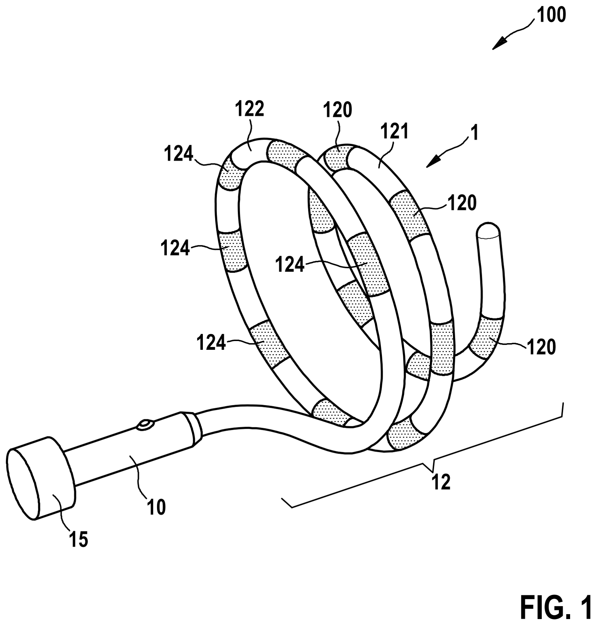

FIG. 1 illustrates a distal portion 100 of an ablation catheter 1 in accordance with a first embodiment. The ablation catheter 1 may be used for PFA, when used with a PFA generator and accessories, and is indicated for use in cardiac electrophysiological mapping (stimulation and recording) and in high-voltage, pulsed-field cardiac ablation. Peak voltages are, for example, without limitation, +/-1 kV to 5 kV with a pulse width of up to 30 µs. Higher peak voltages (e.g. up to 10 kV) may be used provided the pulse duration is correspondingly shorter (e.g. 0.5 µs). The catheter 1 has an elongated circular catheter shaft 10, which may connect with a handle comprising a steering mechanism at a proximal end (not shown). As a result, the ablation catheter 1 may control deflections of the depicted distal portion 100 carrying the ablation electrodes.

At the illustrated distal end of the catheter shaft 10 an ablation portion 12 is arranged, which comprises a plurality of loop sections 121, 122. The concept of loop sections includes embodiments that use continuous loops or spiral configurations. The catheter shaft 10 may have an effective length of approximately 115 cm from the distal tip of the ablation portion 12. Each of a first loop section 121 and a neighboring second loop section 122 exhibits ablation electrodes 120 and mapping electrodes 124 (altogether, for example, 2, 4, 6, 8, 10, 12, 14, 16, or 32 electrodes), which are configured for delivering energy to tissue and receiving electrical signals from tissue. Although two loops are illustrated in FIG. 1, more can be used. It is preferred that at least a partial third loop is used in order to provide sufficient overlap among resulting ablation zones. Said overlap would increase chances of achieving a conduction block moat without drops in lesion continuity, contiguity or transmurality. The distal section comprises at least 45° of overlap of a 3rd loop section with the previous two sections. In particular, the ablation catheter 1 may be configured for delivering an electrical high voltage PFA signal to tissue via the ablation electrodes 120. For example, the ablation electrodes 120 may consist of or comprise gold and/or a platinum/iridium alloy. Alternatively, electrodes 120 from different loop sections may be positioned so that electrodes of same polarity are aligned. However, dependent on the form of the patient's tissue and the position of the ablation portion 12, ablation electrodes 120 of opposite polarities may collide when the spiral catheter is compressed thereby causing arcing and/or the contact of the electrodes with the patient's tissue may not be uniform. In the exemplary embodiment illustrated in FIG. 1, the ablation electrodes 120 of the first loop section 121 are arranged partly in a staggered manner with respect to the ablation electrodes 120 of the second loop section 122 to avoid collision and arcing.

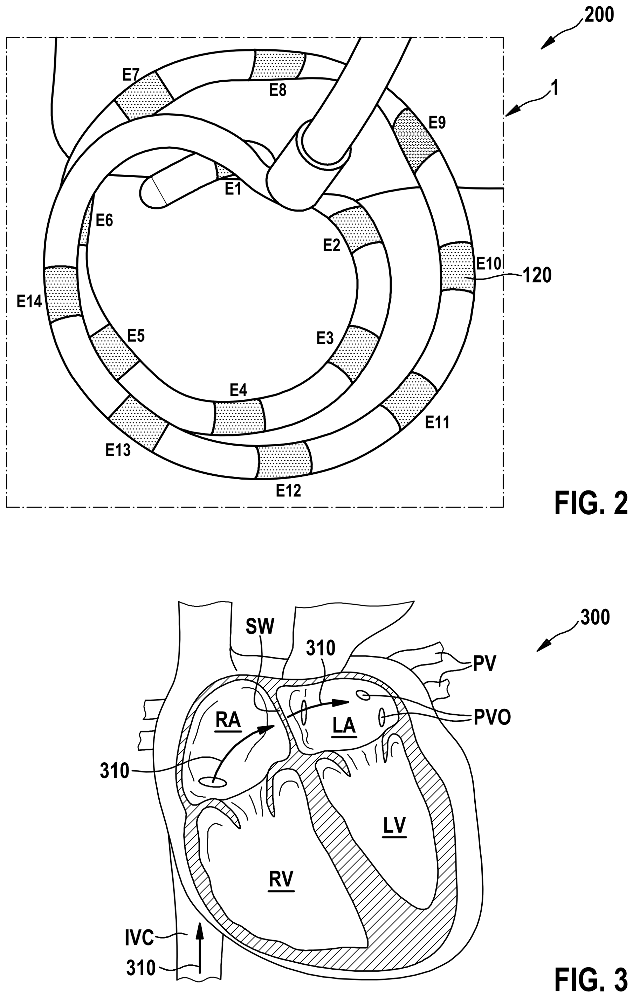

In one aspect, to attribute measurement values to the different electrodes 120, the electrodes are consecutively numbered E1 - E14 as shown, e.g., in FIG. 2 (see numbers at the electrodes). The most distal electrode has the number E1, whereas the most proximal electrode is denoted with number E14. Different numbering is possible, as well.

The loop sections 121, 122 may further exhibit a plurality of mapping electrodes 124, which are configured to receive electrical signals from tissue. In one aspect, mapping electrodes may be configured to receive electrical signals from tissue and deliver the electrical signals to the electronic control unit or the measurement unit. For example, a mapping electrode 124 may receive electrical signals or biopotential signals from vascular tissue or atrial tissue and send those signals to the ECU or the measurement unit for analysis and/or processing. Accordingly, the mapping electrodes may be electrically isolated from the high power pulse field energy source (e.g., PFA generator) that is configured to deliver electrical energy to the ablation electrodes during ablation. Such electrical isolation may advantageously allow the mapping electrodes to operate simultaneously with the ablation electrodes during active ablation.

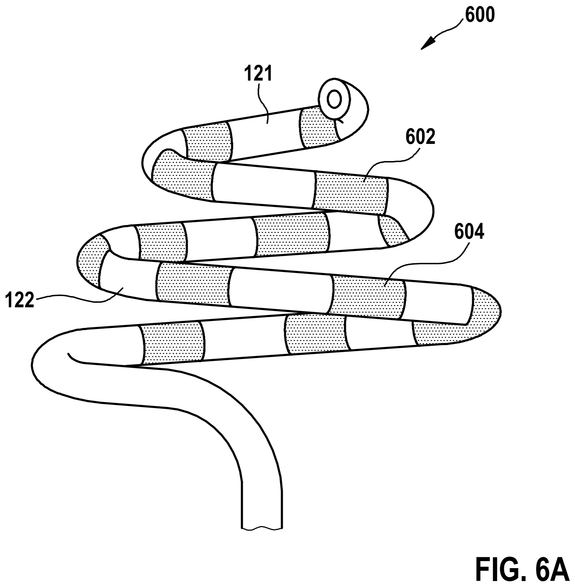

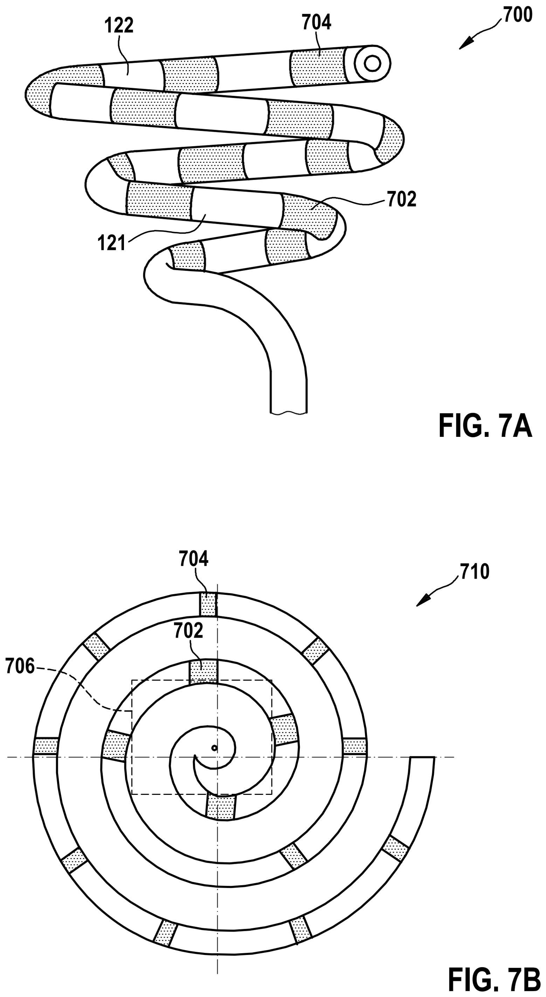

Together, the loop sections 121, 122 form a three-dimensional spiral, which form a corkscrew-like form (e.g., as shown in FIG. 6A) where the diameter of each loop decreases toward the distal end. Alternatively, they may form a plunger-like configuration (e.g., as shown in FIG. 7A) where the diameter of the loops increases toward the distal end or any other suitable 3-dimensional configuration. In either configuration (e.g., corskscrew configuration of FIG. 6A or plunger configuration of FIG. 7A), the innermost portion of the three-dimensional spiral is labeled as the first loop section 121.

The three-dimensional spiral is configured to transition between an uncompressed position and a compressed position. In one aspect, in the uncompressed position, the ablation portion is not restricted by any external force and in the compressed position, the ablation portion is compressed by an external force, e.g., a physician manipulating the handle and/or steering mechanism. For example, in the compressed position, the three-dimensional spiral of the ablation portion 12 may be flattened or substantially flattened by external force, e.g. when the catheter is pressed against tissue by an external force such as a physician manipulating the handle and/or steering mechanism. Accordingly, in the compressed position, the at least two loop sections of the three-dimensional spiral may be substantially in the same plane with respect to a central axis of the three-dimensional spiral. In the compressed position, the at least two loop sections may not be substantially planar, but instead may take on the shape of the corresponding tissue against which each loop section is being pressed. In this aspect, although the at least two loop sections may be contorted under external pressure into a nonplanar configuration, the three-dimensional spiral is still considered to be in the compressed position because the at least two loop sections are pressed into the position by external forces.

The loop sections 121, 122 may comprise a shape memory material, for example, in the form of an inner structural support wire (not illustrated), for example a Nitinol wire as described above. In particular, the loop sections 121, 122 may have super-elastic properties. In one aspect, the inner most loop section, whether in the plunger or corkscrew configuration, comprises a shape memory material that is more stiff than the shape memory material forming the other loop sections.

The ablation portion 12 may be constrained into an essentially elongate shape for the purpose of delivery to a target region in the human body by means of a (fixed or steerable) delivery sheath 15, which may also be referred to as an introducer sheath. At the target position, upon exiting a distal end of the delivery sheath 15, the ablation portion 12 may then recoil to its original (three-dimensional spiral) shape.

The length of each electrode 120 along the respective loop section 121, 122 is, for example, 4 mm. In general, the electrode length is in the range 1-10 mm, preferably 2-5 mm. The catheter shaft 10 size may be compatible with an 8.5 F ID sheath and may consist of radiopaque extrudable polymer and, if applicable, a polymer-reinforcing braid. In general, the size of the catheter shaft 10 may be compatible with a 7 F to 14 F ID sheath. The width between neighboring electrodes along the respective loop section may be chosen between 1 mm and 10 mm, preferably 3-6 mm, in order to provide a contiguous ablated area at the patient's tissue. In one aspect, the distance across the interior of the three-dimensional spiral between ablation electrodes 120 is not greater than 12 mm in the compressed position.

FIG. 3 schematically and exemplarily illustrates a delivery path 300 for an ablation catheter 1 leading to a pulmonary vein ostium (PVO) of a human heart. For orientation, the inferior vena cava (IVC), the right atrium (RA), the right ventricle (RV), the left atrium (LA), the left ventricle (LV), as well as pulmonary veins (PV), each with a PVO, are shown. The large black arrows indicate a delivery path 310 passing through the IVC, the RA, transeptally through the septal wall (SW), and into the LA. Finally, using appropriate deflection means, catheter 1 is steered to PVO regions. There, the corkscrew or plunger type ablation catheter may be used for ablation in the area of the atrial end of the pulmonary vein close to PVO. The form of the ablation portion 12 is configured such that it fits to the dimensions of the targeted PVO. Alternatively, corkscrew or plunger type catheters may be used to ablate at the SVC or at Appendages, such as the left or right atrial appendages (LAA or RAA).

Reliable full ablation along a whole circumference is achieved with the first embodiment of the ablation catheter shown in FIGS. 1 and 2 at their respective position within the heart or the vein to which the form is adapted. A small compression of the ablation portion 12 of the respective catheter 1 may be possible during ablation into the direction of the longitudinal axis of the spiral.

The ablation procedure using one of the ablation catheters 1 may start after the ablation portion 12 is in the correct position relative to the targeted tissue, for example at a PVO. The assessment of the position and/or configuration of the ablation electrodes 120 is provided prior and/or between two ablation steps (if applicable) and is explained in more detail below. The ablation electrodes 120 will provide pulsed electric field in a unipolar or bipolar arrangement. Peak voltages are, for example, without limitation, +/-1 kV to 3 kV with a pulse width of up to 30 µs. Higher peak voltages (e.g. up to 10 kV) may be used provided the pulse duration is correspondingly shorter (e.g. 0.5 µs). The pulse width may be 5 µs (between 0.5-30 µs) forming a pulse train comprising up to 500 pulses/train.

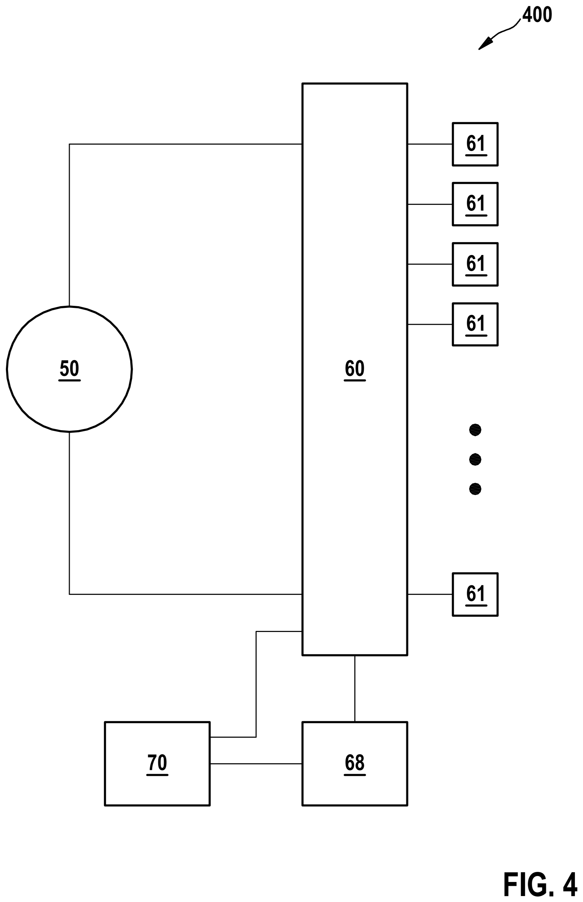

FIG. 4 illustrates an example part 400 of the electric control of the electrode leads 61 for the example ablation catheter of FIG. 1. The electric field generation (in particular voltage, current and impedance) is monitored by an electronic control unit (ECU) 70 which is connected to the leads 61 of the electrodes 120 and produced by a waveform generator 50.

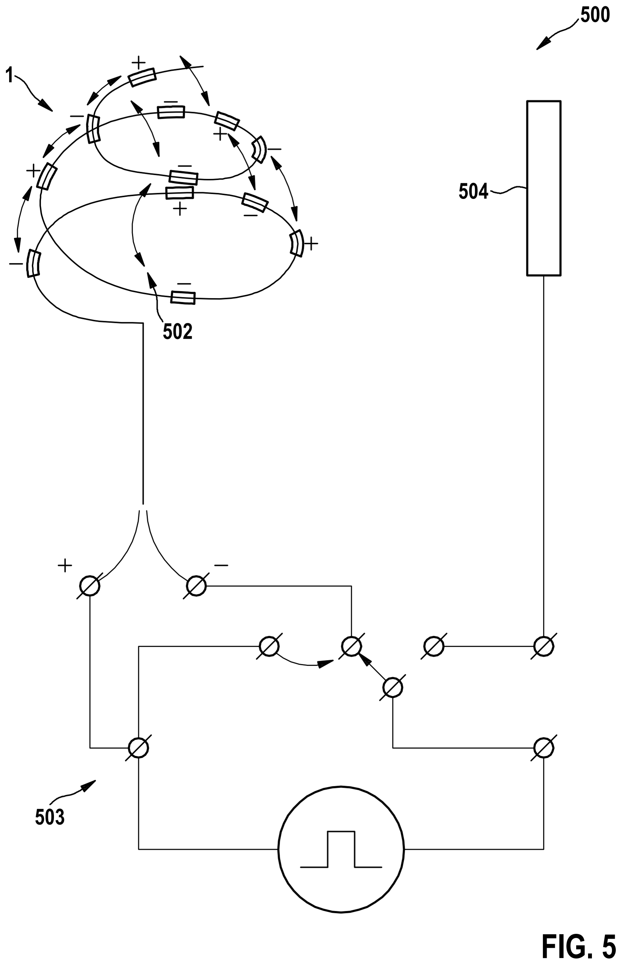

FIG. 5 illustrates an example part 500 of the electric control of the electrode leads for the example ablation catheter of FIG. 1. FIG. 5 also shows connectivity that can be used to generate unipolar or bipolar electric fields. ECUs in FIG. 4 and FIG. 5 may control application of PFA fields. FIG. 5 illustrates a catheter 501 (similar to the catheter with reference number 1 from FIGS. 1 and 2) with its electrodes driven by ECU 503. ECU 503 can be controlled to deliver field vectors 502 that cover the tissue zone in between catheter 501 spiral arms/loops. By doing so, the arcing risk (AR) index may be determined. In order to provide quasi-unipolar measurements, the PFA generator may be connected to one of the electrodes as the reference electrode instead of to the grounding pad 504.

Turning back to FIG. 4, in order to assess the positions and/or configuration of the electrodes 120 with regard to each other and the targeted tissue, the ablation catheter further comprises a measurement unit 68 which is connected to the ECU 70 and a switch unit 60 with the waveform generator 50. The measurement unit 68 is configured to measure peak current and peak voltage as well as impedance at the respective electrode lead 61 and transmit these data to the ECU 70 for further analysis. Further, the measurement unit 68 provides the electrodes 120 at the respective lead(s) 61 with pre-defined measurement signals (current or voltage pulses) via the waveform generator 50 in order to measure the above-mentioned parameter.

In the bipolar arrangement neighboring (adjoining) electrodes 120 may be paired along the loop sections 121, 122, across two neighboring loop sections 121 and 122 or any other pre-defined pair combination, in particular for impedance determination for AR value and/or contact uniformity (CU) value. Further, the electrodes 120 may be used in a unipolar arrangement. In this case, a ground pad 504 may be provided at the surface of the patient's body. Alternatively, one of the non-adjacent electrodes 120 may be used as reference electrode thereby forming a quasi-unipolar arrangement.

In order to switch between different bipolar arrangements or between unipolar and bipolar arrangement, the ablation catheter 1 may comprise a switch unit 60 connected to and controlled by the ECU 70. The switch unit 60 provides the respective phase of the pulsed electric field provided by the waveform generator 50 to the predefined electrode lead 61 and thereby to the predefined electrode 120 wherein each electrode lead 61 is electrically connected to one particular electrode 120 at the ablation portion 12. The switch unit 60 comprises a switch matrix and may realize any configuration of phase distribution, for example, such that two neighboring electrodes along the loop sections, across the loop sections and any other electrodes are paired. The switching signal and configuration information is provided by the ECU 70. ECU 70 further may provide data processing of electrical or biopotential data or impedance data acquired the electrodes of ablation catheter 1. As indicated above mapping electrodes 124 located in the ablation portion 12 may comprise mapping electrodes for determining the electrical potential of the surrounding tissue in order to observe the ablation progress at pre-defined time points during the ablation procedure. Alternatively, the ablation electrodes 120 may be switched into the mapping mode and back into the ablation mode.

Advantageously, prior to ablation treatment and/or between ablation treatment steps the AR value and CU value are determined in order to assess the positions of the electrodes 120 and/or their configuration with regard to each other and/or with regard to the tissue under treatment.

In one aspect, the AR value is calculated using the formula: ${AR}_{x , y} = 1 - \frac{{Z}_{x , y}}{min \left({Z}_{x - 1 , x}, {Z}_{x , x + 1}, {Z}_{y - 1 , y}, {Z}_{y , y + 1}\right)}$

In one aspect, the calculated AR values are zero or close to zero when the ablation portion of the catheter is uncompressed and measured in saline. No risk of arcing exists since all electrodes have a sufficient distance between each other in the uncompressed position. In contrast, when electrodes of opposite polarities are close to each other, e.g., in the compressed position, there is an arcing risk with regard to these electrodes and repositioning may be needed.

In one aspect, the CU value is calculated using the below formula and the measured bipolar impedances of the adjoining electrodes: $CU = 1 - \frac{σ \left(\left\{{Z}_{n , n + 1}\right\}\right)}{μ \left(\left\{{Z}_{n , n + 1}\right\}\right)}$

The calculated contact uniformity value is best in the saline configuration (e.g., 0.99) because all electrodes are without contact, i.e. all electrodes are floating in saline. In practice, when the electrodes of the catheter are in good and uniform contact with the target tissue in the PVO, the CU value is high, i.e., approaching 1. On the other hand, the CU value decreases when the tissue contact becomes non-uniform, i.e., when some electrodes are in good contact, while others are in poor contact or are entirely in the blood pool. In one aspect, displaying the CU value on a graphical user interface (GUI) and periodically updating the displayed value (e.g., per second, per PFA shot, per measurement cycle, etc.) may help the physician to decide whether to reposition the catheter in an easily understandable, reliable and time-effective way.

FIG. 6A illustrates a side view of an example ablation portion 600 of an ablation catheter arranged in a corkscrew embodiment. In the illustrated embodiment, the ablation catheter is in an uncompressed position and includes a plurality of ablation electrodes 602 at the distal end of the ablation portion 600 of the ablation catheter such that the ablation electrodes 602 are positioned on the inner most loop (first loop section 121) of the three-dimensional spiral. The ablation catheter also includes a plurality of mapping electrodes 604 at the proximal end of the ablation portion 600 of the ablation catheter (second loop section 122) such that the mapping electrodes 604 are positioned more externally on the three-dimensional spiral than the ablation electrodes relative to a central axis of the three-dimensional spiral.

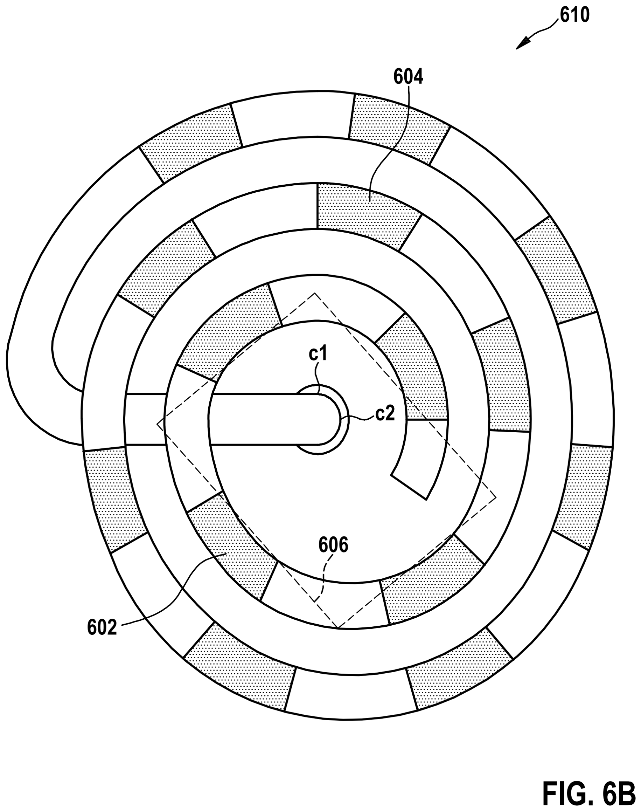

FIG. 6B illustrates a plan view of an example ablation portion 610 of an ablation catheter arranged in a corkscrew embodiment. In the illustrated embodiment, the ablation catheter is in a compressed position and includes a plurality of ablation electrodes 602 at the distal end of the ablation portion 610 of the ablation catheter such that the ablation electrodes 602 are positioned on the inner most loop of the three-dimensional spiral. The ablation catheter also includes a plurality of mapping electrodes 604 at the proximal end of the ablation portion 610 of the ablation catheter such that the mapping electrodes 604 are positioned more externally on the three-dimensional spiral than the ablation electrodes.

As shown in the plan view, there are four ablation electrodes 602 and when the ablation portion 610 is in the compressed position, each ablation electrode 602 has an edge that faces the interior of the three-dimensional spiral formed by the ablation portion 610. Additionally, the edges of a first pair of ablation electrodes that face each other across the interior of the three-dimensional spiral are substantially parallel to each other and substantially perpendicular to the edges of the second pair of other two ablation electrodes that face each other across the interior of the three-dimensional spiral. In this configuration, the four ablation electrodes 602 are positioned in a rectangular arrangement 606. In one aspect, the lengths of the sides of the rectangular arrangement 606 substantially form a square.

FIG. 7A illustrates a side view of an example ablation portion 700 of an ablation catheter arranged in a plunger embodiment. In the illustrated embodiment, the ablation catheter is in an uncompressed position and includes a plurality of ablation electrodes 702 at the proximal end of the ablation portion 700 of the ablation catheter such that the ablation electrodes 702 are positioned on the inner most loop (first loop section 121) of the three-dimensional spiral. The ablation catheter also includes a plurality of mapping electrodes 704 at the distal end of the ablation portion 700 of the ablation catheter (second loop section 122) such that the mapping electrodes 704 are positioned more externally on the three-dimensional spiral than the ablation electrodes relative to a central axis of the three-dimensional spiral.

FIG. 7B illustrates a plan view of an example ablation portion 710 of an ablation catheter arranged in a plunger embodiment. In the illustrated embodiment, the ablation catheter is in a compressed position and includes a plurality of ablation electrodes 702 at the distal end of the ablation portion 710 of the ablation catheter such that the ablation electrodes 702 are positioned on the inner most loop of the three-dimensional spiral. The ablation catheter also includes a plurality of mapping electrodes 704 at the proximal end of the ablation portion 710 of the ablation catheter such that the mapping electrodes 704 are positioned more externally on the three-dimensional spiral than the ablation electrodes.

As shown in the plan view, there are four ablation electrodes 702 and when the ablation portion 710 is in the compressed position, each ablation electrode 702 has an edge that faces the interior of the three-dimensional spiral formed by the ablation portion 710. Additionally, the edges of a first pair of ablation electrodes that face each other across the interior of the three-dimensional spiral are substantially parallel to each other and substantially perpendicular to the edges of the second pair of other two ablation electrodes that face each other across the interior of the three-dimensional spiral. In this configuration, the four ablation electrodes 702 are positioned in a rectangular arrangement 706. In one aspect, the lengths of the sides of the rectangular arrangement 706 substantially form a square.

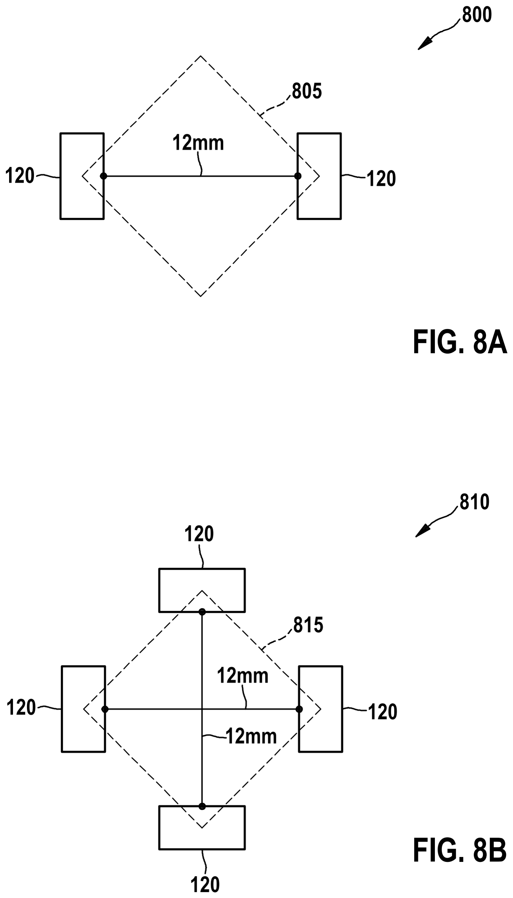

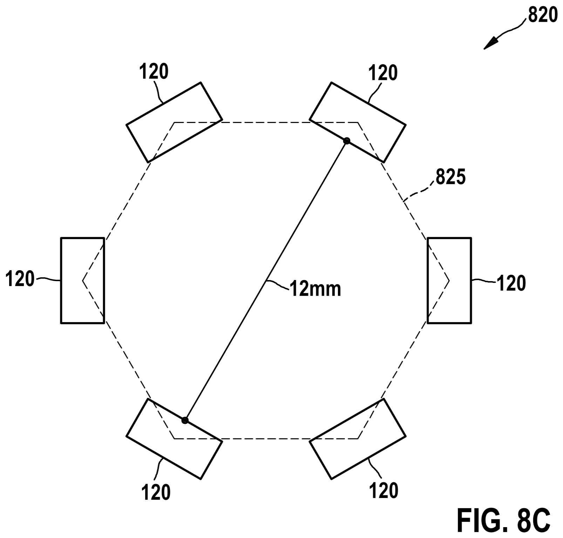

FIGS. 8A, 8B, and 8C illustrate plan view examples of alternative configurations 800, 810, and 820 of the ablation electrodes 120 on the inner-most spiral of the ablation portion of the ablation catheter.

In FIG. 8A, there are two ablation electrodes 120 that face each other across the interior of the three-dimensional spiral of the ablation portion. In one aspect, each of the two ablation electrodes 120 occupies an opposing corner of a rectangle 805 in a plan view in the configuration 800. Likewise, the ablation electrodes could also occupy the side, in particular the center area of a side of the rectangle. In one aspect, the rectangle is a square. In one aspect, the distance between the facing edges of the two ablation electrodes 120 in configuration 800 is not greater than 12mm. In operation, each of the two electrodes 120 produces a respective ablation region when energy is applied to tissue through the respective ablation electrode 120. Advantageously, in configuration 800, the resulting ablation regions from the two ablation electrodes 120 overlap to form a single contiguous ablation region.

In FIG. 8B, there are four ablation electrodes 120. In the illustrated embodiment, the four ablation electrodes 120 form two pairs of ablation electrodes 120, with each ablation electrode 120 in a pair having an interior edge that faces the interior edge of the other ablation electrode 120 other across the interior of the three-dimensional spiral of the ablation portion. In one aspect, each of the four ablation electrodes 120 occupies an opposing corner of a rectangle 815 in a plan view in the configuration 810. Likewise, the ablation electrodes could also occupy the side, in particular the center area of a side of the rectangle. In one aspect, the rectangle is a square. In one aspect, the distance between the facing edges of the two ablation electrodes 120 of any pair of ablation electrodes 120 in configuration 810 is not greater than 12mm. In operation, each of the electrodes 120 produces a respective ablation region when energy is applied to tissue through the respective ablation electrode 120. Advantageously, in configuration 810, the resulting ablation regions from the four ablation electrodes 120 overlap to form a single contiguous ablation region.

In FIG. 8C, there are six ablation electrodes 120. In the illustrated embodiment, the six ablation electrodes 120 form three pairs of ablation electrodes 120, with each ablation electrode 120 in a pair having an interior edge that faces the interior edge of the other ablation electrode 120 of the pair other across the interior of the three-dimensional spiral of the ablation portion. In one aspect, each of the six ablation electrodes 120 occupies a corner of a hexagon 825 in a plan view in the configuration 820. Likewise, the ablation electrodes could also occupy the side, in particular the center area of a side of the hexagon. In one aspect, the distance between the facing edges of the two ablation electrodes 120 of any pair of ablation electrodes 120 in configuration 820 is not greater than 12mm. In operation, each of the electrodes 120 produces a respective ablation region when energy is applied to tissue through the respective ablation electrode 120. Advantageously, in configuration 820, the resulting ablation regions from the six ablation electrodes 120 overlap to form a single contiguous ablation region.

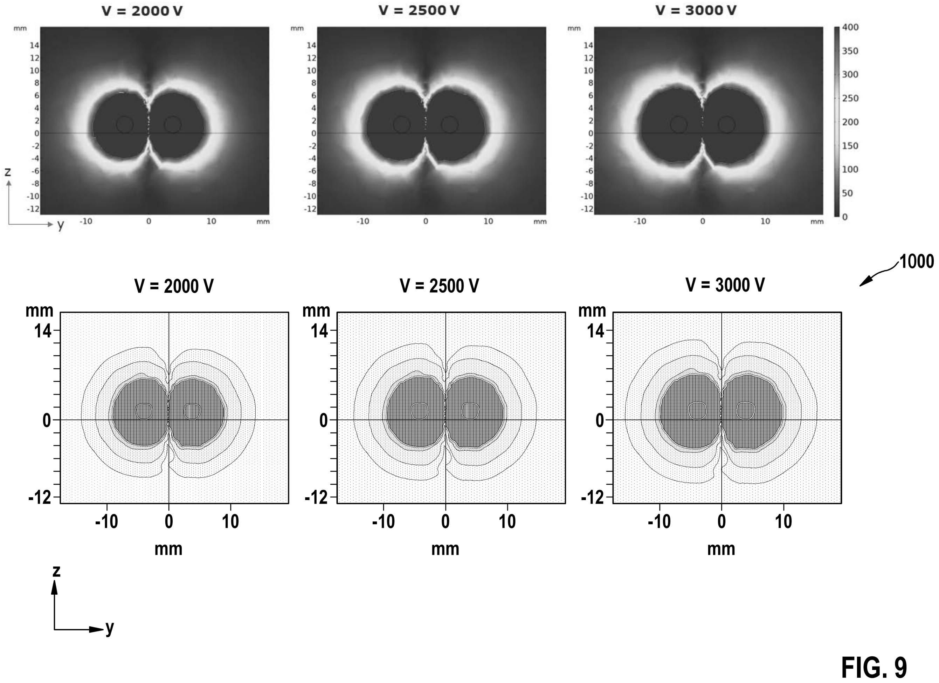

FIGs 9 to 19 illustrate example charts showing lesion extensions at various voltages when energy is applied to the ablation electrodes in a bipolar arrangement. The dimensions of the presentations are not to scale and intend to show that higher voltages create larger lesions.

FIG. 9 illustrates an example chart 1000 illustrating example lesion depth for a 1^{st} example of an ablation catheter at various voltages when energy is applied to the ablation electrodes in a bipolar arrangement. The illustrated lesion depth at each voltage confirms the contiguous ablation region. The example lesion depths are calculated (e.g. simulated) using a finite element method (FEM). The lesion depth is shown as cut perpendicular to the tissue surface. The 1^{st} example of an ablation catheter may comprise a loop as described above having a diameter of 7.64 mm. The loop comprises four electrodes having a length of 2mm whereby the spacing between two adjacent electrodes is 4 mm, e.g. the ablation electrodes are positioned 4mm across the interior of the three-dimensional spiral in the compressed position.

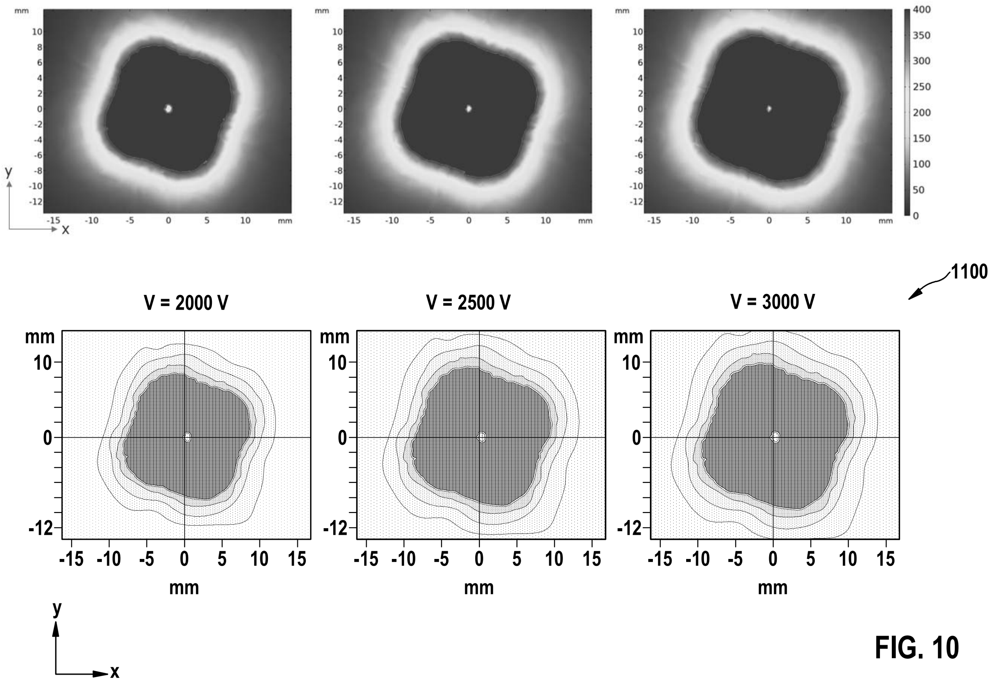

FIG. 10 illustrates an example chart 1100 illustrating example lesion width at 2 mm depth for the ablation catheter configuration of FIG. 9 at various voltages when energy is applied to the ablation electrodes in a bipolar arrangement. The lesion width is shown as cut parallel to the tissue surface. The illustrated lesion width at each voltage confirms the contiguous ablation region. The example lesion widths are calculated using FEM.

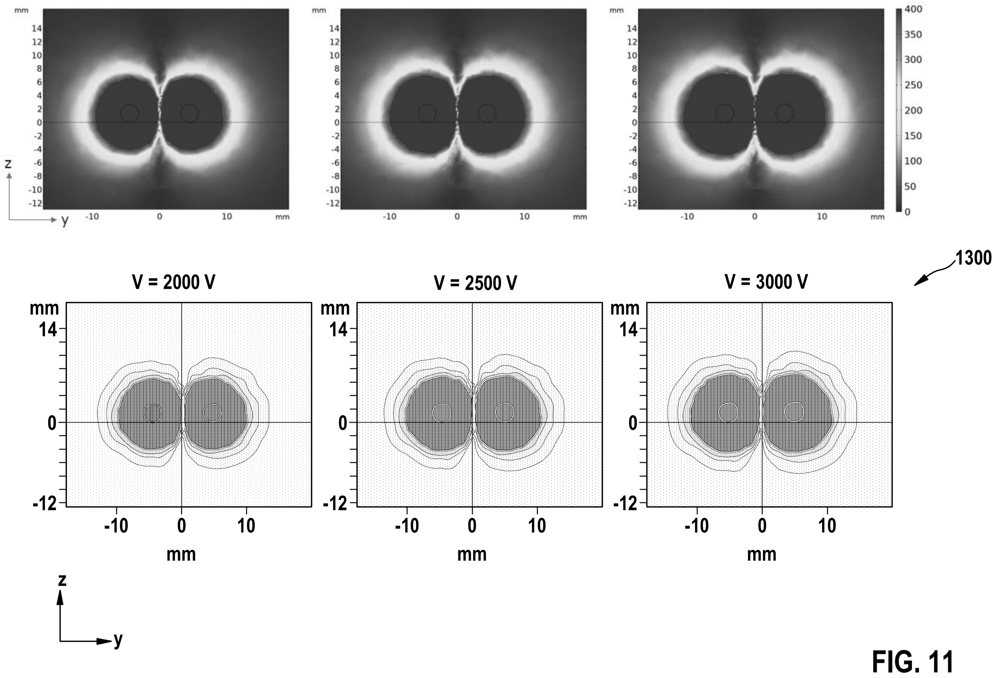

FIG. 11 illustrates an example chart 1300 illustrating example lesion depth for a 2^{nd} example of an ablation catheter at various voltages when energy is applied to the ablation electrodes in a bipolar arrangement. The illustrated lesion depth at each voltage confirms the contiguous ablation region. The example lesion depths are calculated (e.g. simulated) using FEM. The lesion depth is shown as cut perpendicular to the tissue surface. The 2^{nd} example of an ablation catheter may comprise a loop as described above having a diameter of 8.92 mm. The loop comprises four electrodes having a length of 2mm whereby the spacing between two adjacent electrodes is 5 mm, e.g. the ablation electrodes are positioned 5 mm across the interior of the three-dimensional spiral in the compressed position.

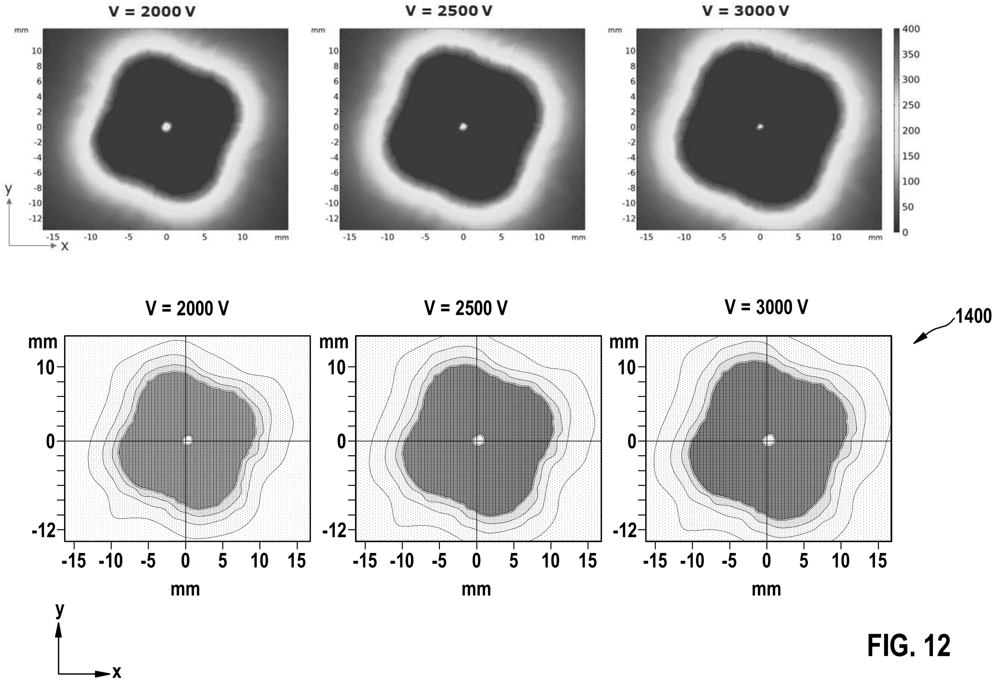

FIG. 12 illustrates an example chart 1400 illustrating example lesion width at 2 mm depth for the ablation catheter configuration of FIG. 11 at various voltages when energy is applied to the ablation electrodes in a bipolar arrangement. The lesion width is shown as cut parallel to the tissue surface. The illustrated lesion width at each voltage confirms the contiguous ablation region.

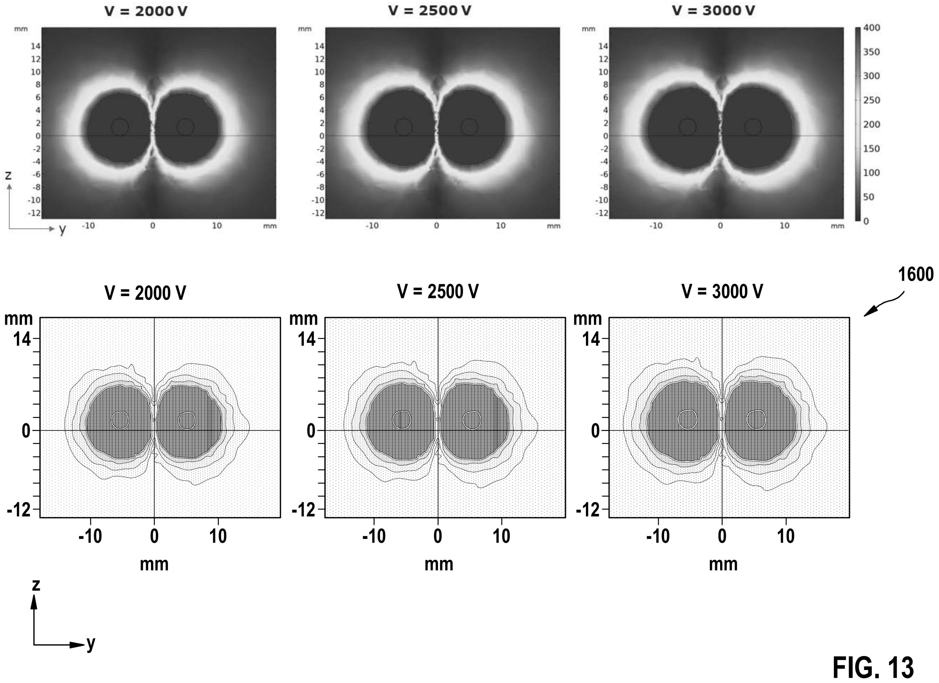

FIG. 13 illustrates an example chart 1600 illustrating example lesion depth for a 3^{rd} example of an ablation catheter at various voltages when energy is applied to the ablation electrodes in a bipolar arrangement. The illustrated lesion depth at each voltage confirms the contiguous ablation region. The example lesion depths are calculated (e.g. simulated) using FEM. The lesion depth is shown as cut perpendicular to the tissue surface. The 3^{rd} example of an ablation catheter may comprise a loop as described above having a diameter of 10.2 mm. The loop comprises four electrodes having a length of 2mm whereby the spacing between two adjacent electrodes is 6 mm, e.g. the ablation electrodes are positioned 6 mm across the interior of the three-dimensional spiral in the compressed position.

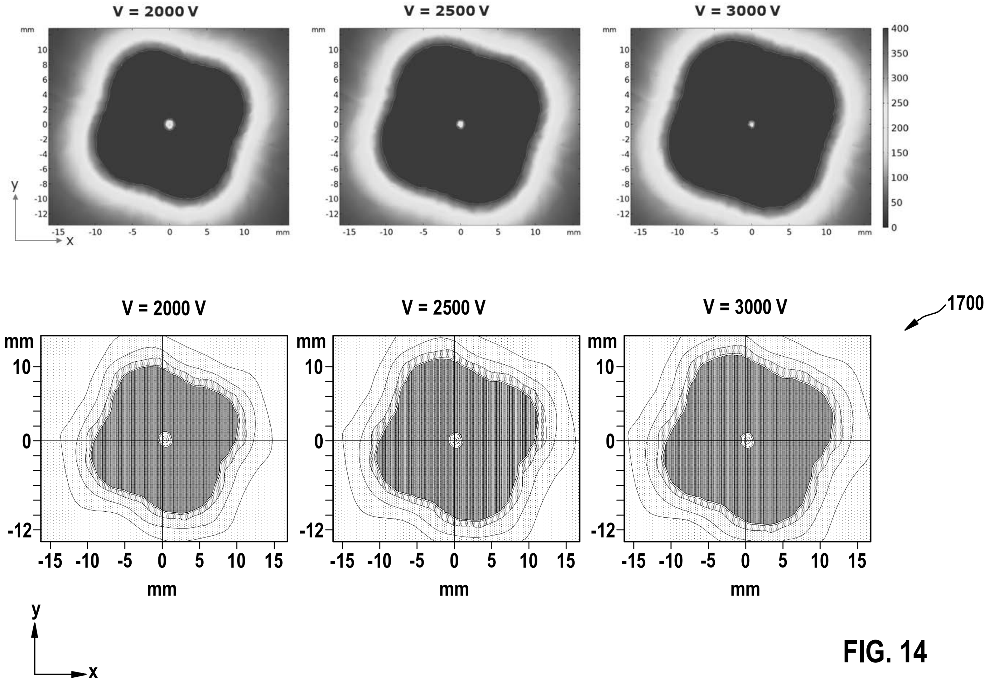

FIG. 14 illustrates an example chart 1700 illustrating example lesion width at 2 mm depth for the ablation catheter configuration of FIG. 13 at various voltages when energy is applied to the ablation electrodes in a bipolar arrangement. The lesion width is shown as cut parallel to the tissue surface. The illustrated lesion width at each voltage confirms the contiguous ablation region.

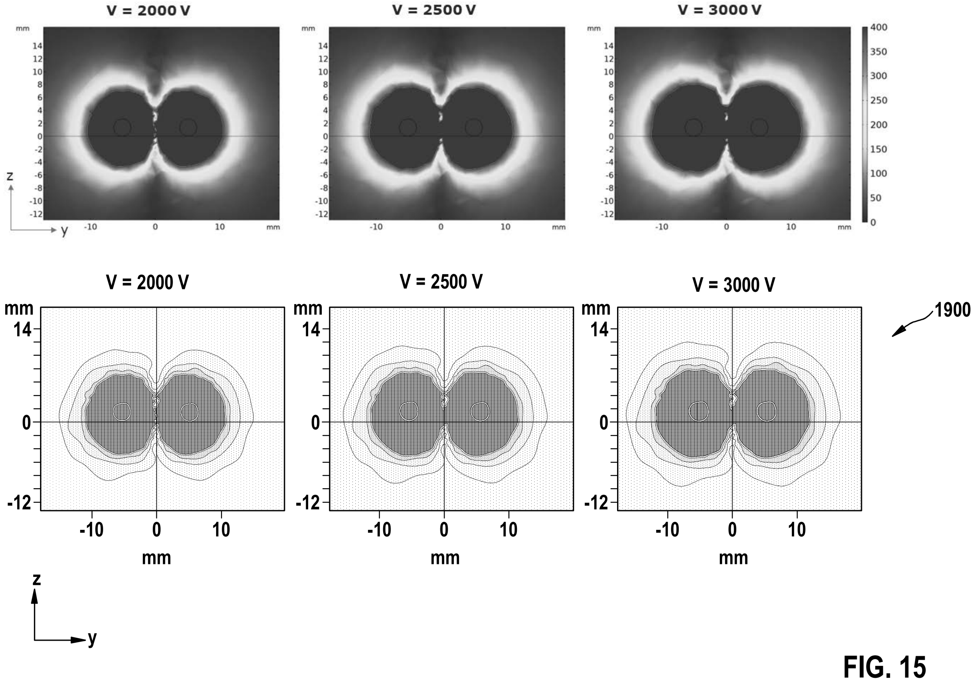

FIG. 15 illustrates an example chart 1900 illustrating example lesion depth for a 4^{th} example of an ablation catheter at various voltages when energy is applied to the ablation electrodes in a bipolar arrangement. The illustrated lesion depth at each voltage confirms the contiguous ablation region. The example lesion depths are calculated (e.g. simulated) using FEM. The lesion depth is shown as cut perpendicular to the tissue surface. The 4^{th} example of an ablation catheter may comprise a loop as described above having a diameter of 10.2 mm. The loop comprises four electrodes having a length of 3 mm whereby the spacing between two adjacent electrodes is 5 mm, e.g. the ablation electrodes are positioned 5 mm across the interior of the three-dimensional spiral in the compressed position.

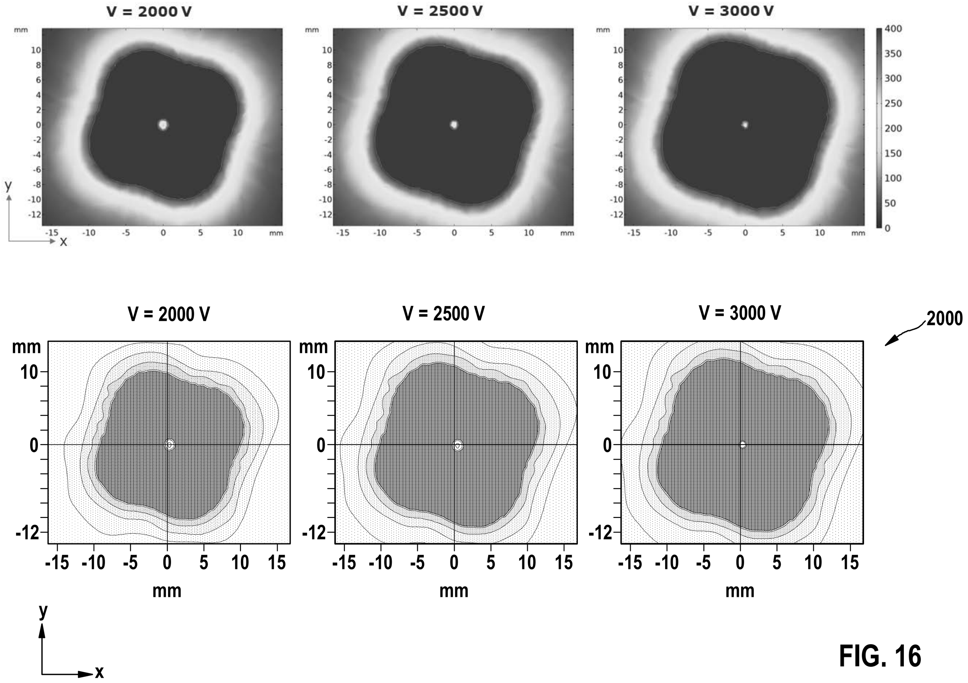

FIG. 16 illustrates an example chart 2000 illustrating example lesion width a 2 mm depth for the ablation catheter configuration of FIG. 15 at various voltages when energy is applied to the ablation electrodes in a bipolar arrangement. The lesion depth is shown as cut parallel to the tissue surface. The illustrated lesion width at each voltage confirms the contiguous ablation region.

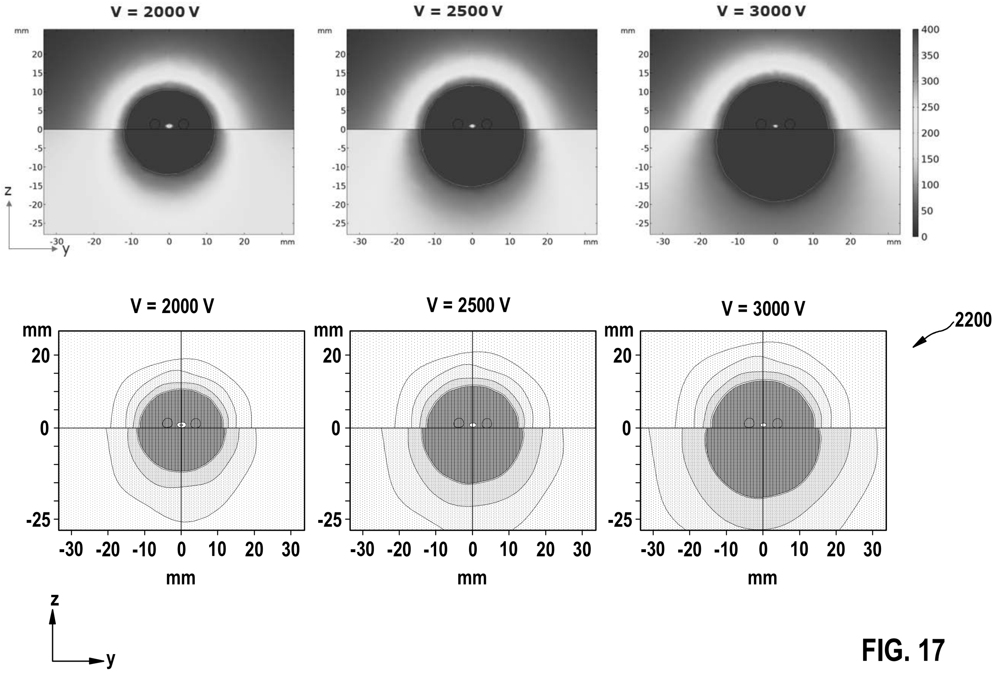

FIG. 17 illustrates an example chart 2200 illustrating example lesion depth for a 5^{th} example of an ablation catheter at various voltages when energy is applied to the ablation electrodes in a unipolar arrangement. The illustrated lesion depth at each voltage confirms the contiguous ablation region. The example lesion depths are calculated (e.g. simulated) using FEM. The lesion depth is shown as cut perpendicular to the tissue surface. The 5^{th} example of an ablation catheter may comprise a loop as described above having a diameter of 7.6 mm. The loop comprises four electrodes having a length of 2 mm whereby the spacing between two adjacent electrodes is 4 mm, e.g. the ablation electrodes are positioned 4 mm across the interior of the three-dimensional spiral in the compressed position

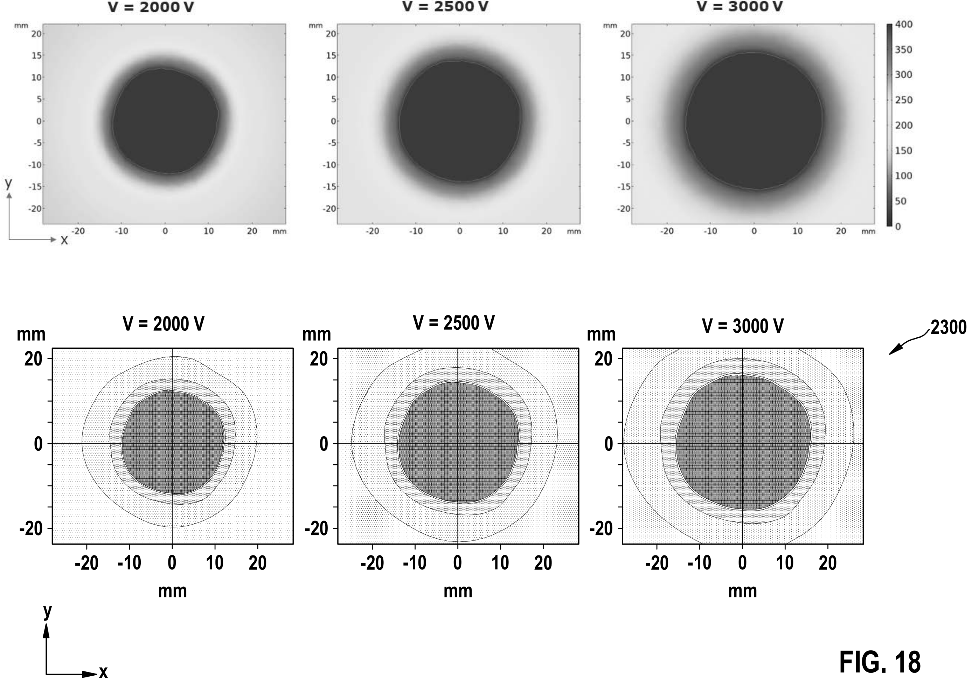

FIG. 18 illustrates an example chart 2300 illustrating example lesion width at 2mm depth for the ablation catheter configuration of FIG. 17 at various voltages when energy is applied to the ablation electrodes in a unipolar arrangement. The lesion depth is shown as cut parallel to the tissue surface. The illustrated lesion depth at each voltage confirms the contiguous ablation region.

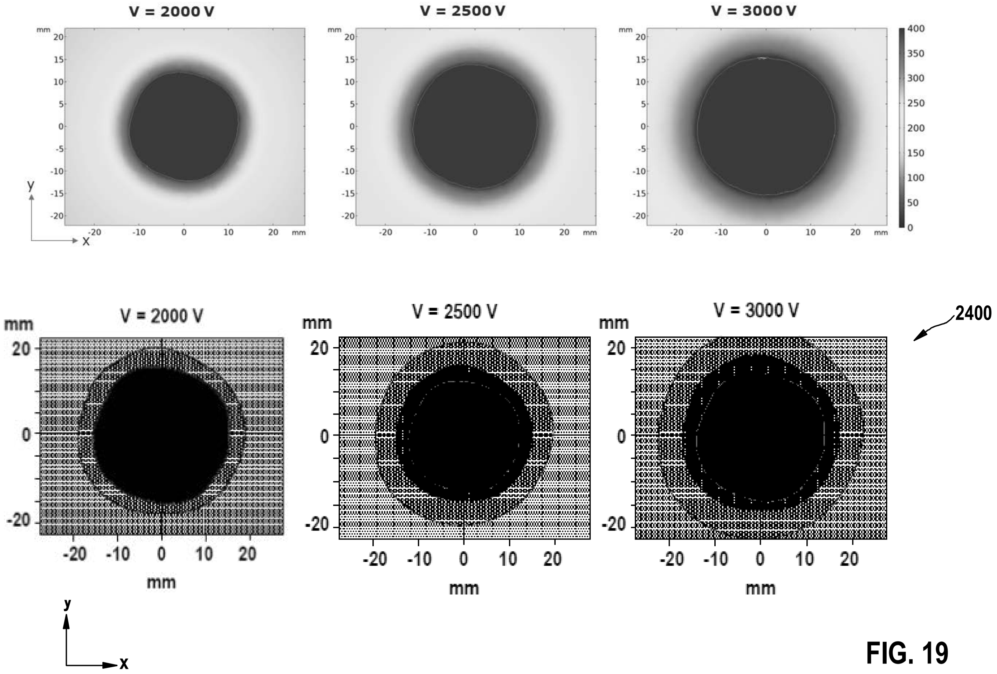

FIG. 19 illustrates an example chart 2400 illustrating example lesion width at 0.5mm depth for the ablation catheter configuration of FIG. 17 at various voltages when energy is applied to the ablation electrodes in a unipolar arrangement. The lesion depth is shown as cut parallel to the tissue surface. The illustrated lesion depth at each voltage confirms the contiguous ablation region.

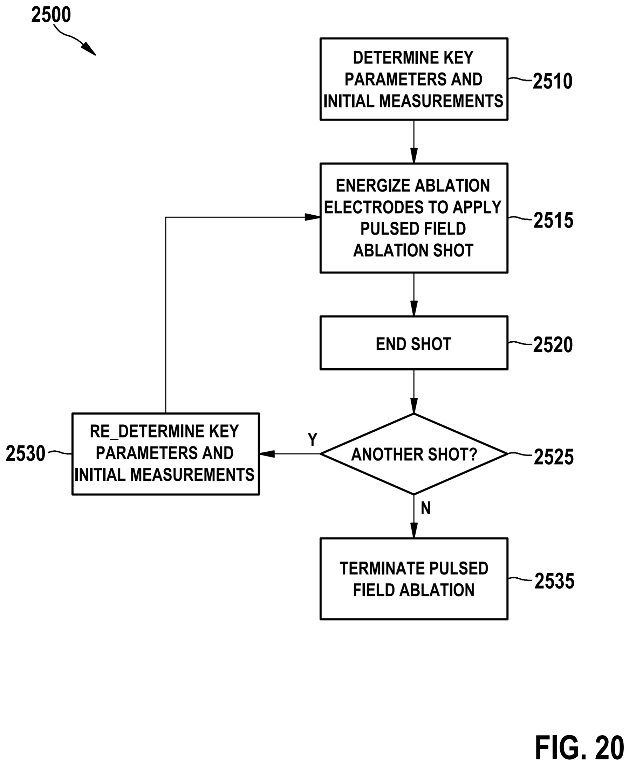

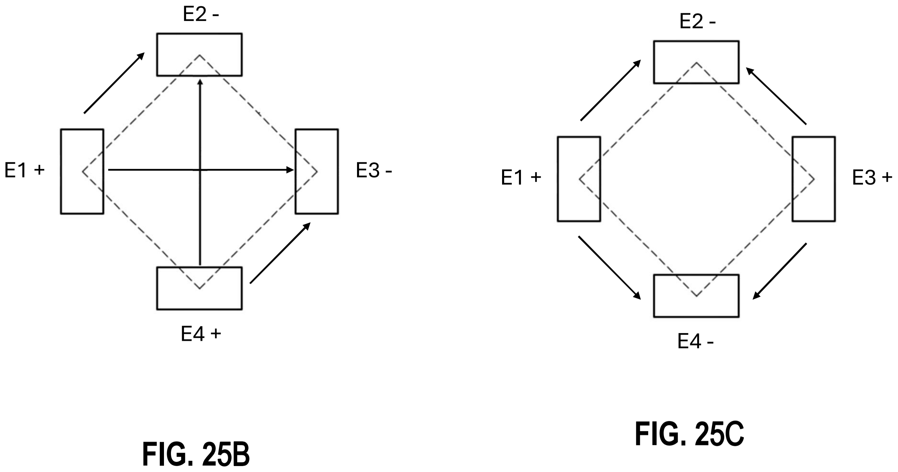

FIG. 20 is a flow diagram illustrating an example process for treatment of patient tissue by delivery of high-voltage pulses in a bipolar or unipolar arrangement. In one aspect, the process of FIG. 25 may be carried out by the ablation catheter systems described with respect to FIGS. 1-8C.

Initially, at 2510, certain key parameters and initial measurements are determined by the ECU. For example, certain key parameters such as the pulse protocol (e.g., number of pulses, pulse width, and pulse separation), applied pulse voltage, the number of pulses in each PFA pulse train, energy delivery using bipolar or unipolar arrangement, catheter dimensions (e.g., number of electrodes, electrode positions, and electrode spacings), which electrodes to use when delivering energy to tissue, and patient information (e.g., height, weight, hematocrit value, GFR, and blood volume) may be received by the ECU via a user interface. Additionally, certain measurements such as the impedance values at each electrode may be taken to calculate the arcing risk and the contact uniformity for the electrodes that will be activated during delivery of energy to tissue. Certain parameters may also be calculated or obtained from a memory where standard values are stored or received via a user interface.

Next, at 2515, the ECU applies the first PFA pulse train in a single PFA shot by delivering energy to tissue via one or more electrodes using the selected bipolar arrangement or unipolar arrangement.

Next, at 2520, when the single PFA shot is completed, the ECU ends the delivery of energy to tissue. If another PFA shot is needed, as determined at 2525, the ECU re-determines the key parameters and initial measurements at 2530 and loops back to 2515 to apply the next PFA shot. If another PFA shot is not needed, as determined at 2525, the PFA treatment is terminated at 2535.

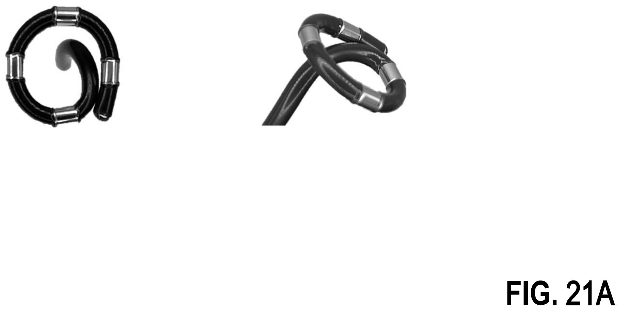

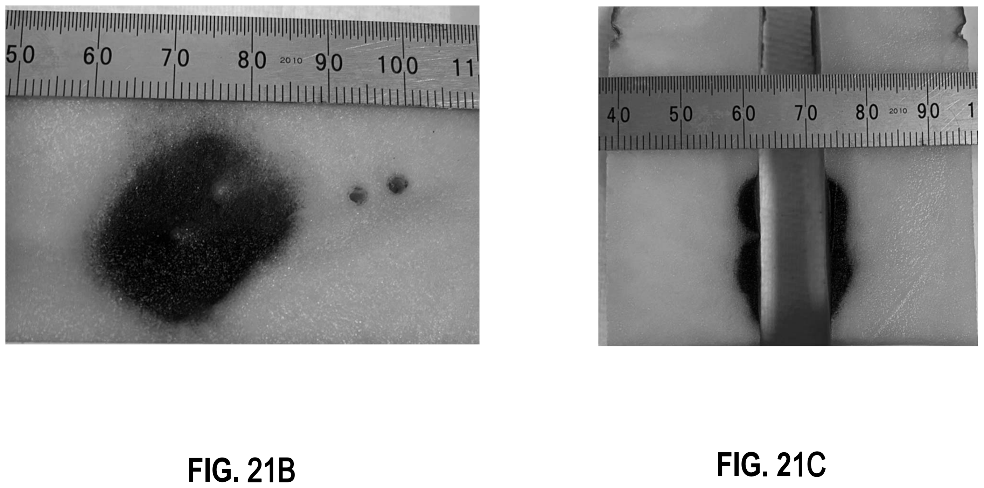

FIG. 21 A shows an exemplary electrode arrangement on an ablation catheter according to the present invention. The catheter body was fixed into a spiral wherein the four electrode are arranged in 12, 3, 6 and 9 o'clock positions. The distance between the 12 and 6 o'clock positions electrode's inner edges are approx. 9 mm, while the distance between the 3 and 9 o'clock position's inner edges are about 10.5 mm. For the ablation study on a potato the four electrodes are provided with corresponding current.

On FIG.s 21 B and C it can be seen that the ablation takes place very focused and results in focused lesion with width and breadth of each approx. 2.5 cm. The depth of the ablation is also focused and up to 4 mm.

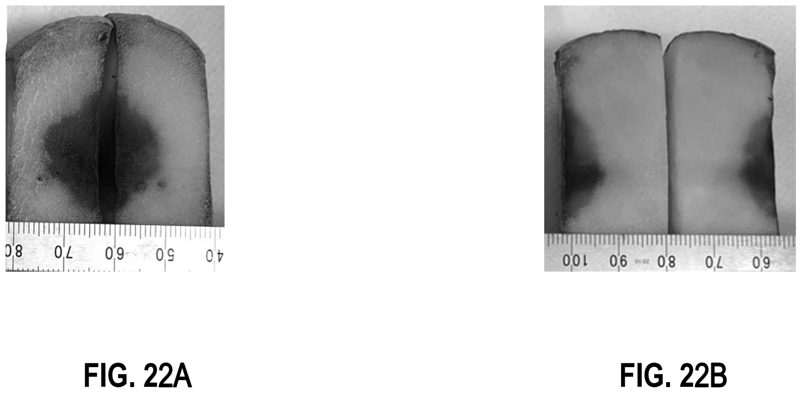

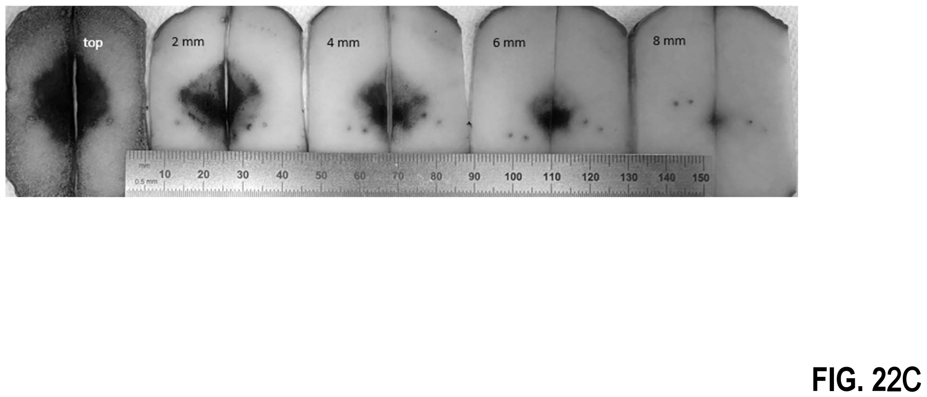

Moreover, FIGs 22 A-C shows results on potatoes of an ablation procedure using two different electrode configurations as summarized in the tables of FIGs 25 A-C. For the procedures the catheter was applied perpendicular to the potato. Energy was applied according to configurations shown in Figs. 25A or 26A and as described in the patent applications WO 2022/159659 A1 and WO 2023/150215 A1. The potatoes were left to cure for approx. 24 h. Lesion depth and width were then measured using rulers shown in respective figures. It is important to note in view of Figs. 21C and 22B that applying the configurations of Figs 25A and 26A the lesion depth was sufficient for treating focal cardiac disturbances. For the results shown in FIGs 22 in a first pulse train the configuration of FIG. 25 B was applied, followed by a pulse train according to the configuration of FIG. 25 C. It is clear to the skilled person that combinations of the configurations shown in FIGs 25 A for catheters with four electrodes as well as for catheters with six electrodes as shown in FIG. 26 A can be combined in any manner. It is also conceivable that configurations can be used more than once in one ablation procedure and can be combined with any other configuration or configurations. It is also within the scope of the present invention that other catheter arrangements with two, three, five, seven, eight or even more electrodes can be used for an ablation procedure. Within the present invention the distance between the electrodes shall not be greater than 12 mm in pressed down or compressed position as described herein.

In FIGs 22 A and 22 B the ablation result using the four electrodes catheter according to the invention as described herein (as of FIG. 21 A) is shown. A continuous lesion of approx. 9 mm depth and over 2 cm breadth has been generated. In length the lesion has an extent up to 1 cm. Moreover, in FIG 22 C the depth is illustrated in layers of 2 mm thickness. On the left hand side the appearance of the top surface is shown. Going from there to the right for every following picture 2 mm layers have been cut off the potato showing that the lesion is of cone shape and exhibits even at a depth of 6 mm a very thorough (deep black) and focused ablation.

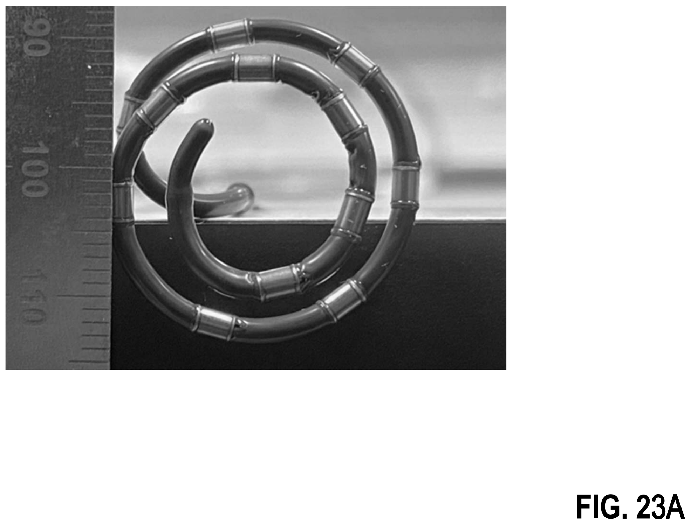

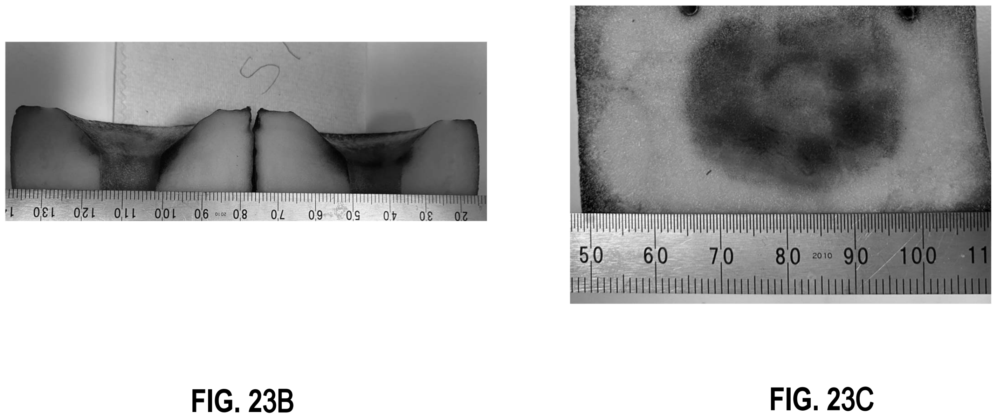

In contrast and comparative, in FIG. 23 A the electrodes of a similar, but different ablation catheter are not fixed in to a 12, 3, 6 and 9 o'clock arrangement, but in a 12, 1,5, 3 and 5 o'clock arrangement. In addition, the distance of the electrodes exceeds 12 mm. The different arrangement of the inner electrodes have a dramatic influence on the ablation results, for which only the four distal electrodes were used. Iin FIG.s 23 B and C, it can be seen that the lesions are shallow and neither focused nor contiguous. The lesion exhibits almost a donut-like structure having a significant bright area in the middle of the lesion of FIG. 23 C. It can easily be deduced from comparison of both ablations that with arrangement according to the invention an ablation procedure will be much shorter and may (if at all) less be repeated in order to achieve a thorough tissue electroporation.

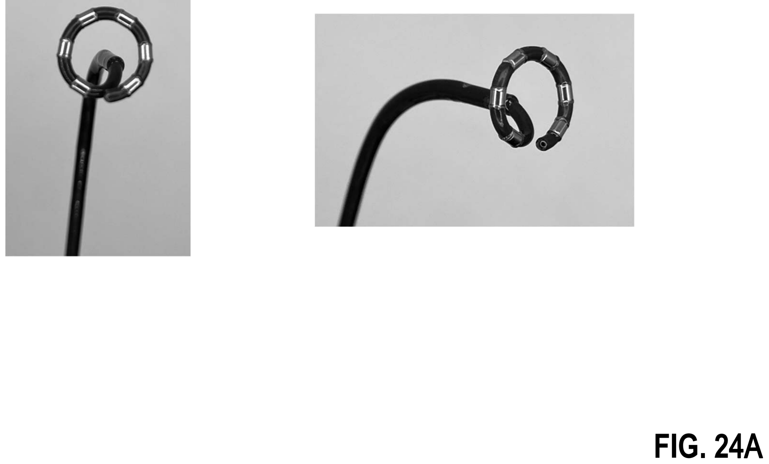

In FIG. 24 A shows an exemplary electrode arrangement on an ablation catheter according to the present invention. The catheter body has a fixed into a fixed arrangement of six electrodes in 11, 1,3, 5, 7 and 9 o'clock positions. The distance between the 9 and 3 o'clock positions electrode's inner edges are less than 12 mm, which also holds true for the other diametrically arranged electrodes.

In FIG. 25A a table is shown summarizing the different possibilities of operating the different electrodes according to FIG. 8B and as exemplary shown in FIG. 21 A. Accordingly, there is in each case two electrodes have a "+" polarity and two other electrodes having a "-" polarity. By operating the different electrodes with different polarities, the current flow of the pulses can be controlled. The position for the electrodes 1 to 4 can be seen in FIGs 25B and C. The current of the pulses is indicated by arrows. Accordingly, current flows from the positively charged electrodes to the negatively charged electrode. For the configuration of FIG. 25 B current in particular flows crosswise through the center of the electrode arrangement. Additionally current flows at two sides of the rectangle. For the configuration of FIG. 25C the current does not cross through the middle of the electrode arrangement, but flows along each side of the rectangle. Combining these to configurations a thorough ablation through the middle as well as along the sides of the rectangle can be achieved as exemplary shown in FIGs A-C.

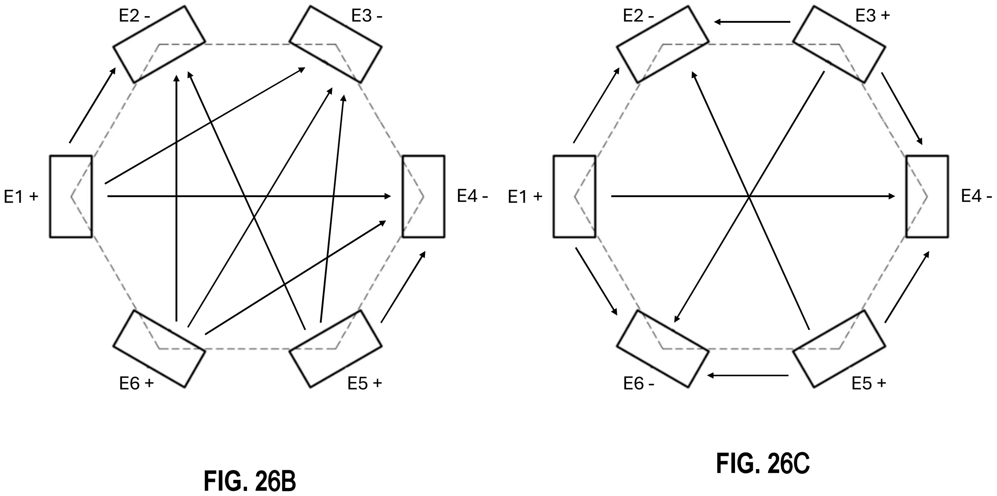

A similar table and currently flow map is provided in FIGs 26 A-C for hexagon arrangement with corresponding six electrodes. The table of FIG. 26 A summarizes the configuration possibilities when three electrodes shall have a positive polarity and the three other electrodes exhibit a negative polarity. As can be seen from FIG. 26 B a strong impact of ablating the center can be deduced from the configuration of FIG. 26B. The configuration of FIG. 26 C provides both center and outside ablation to similar degree. Thereby, the specific needs for ablating a patient's tissue can be addressed by choosing specifically a fitting configuration or as mentioned above a combination of configuration. With advantage, a personalized treatment can therefore be provided with the catheter configurations as described herein.

The disclosure contains the following numbered embodiments:
1. An ablation catheter for treatment of patient tissue by delivery of high-voltage pulses comprising: a catheter shaft having an ablation portion at a distal end of the catheter shaft, wherein the ablation portion comprises at least one, optionally two loop sections forming a round structure or a three-dimensional spiral, wherein optionally the at least two loop sections configured to transition between an uncompressed position where the ablation portion is not restricted by any external force and a compressed position where the ablation portion is compressed by an external force when the catheter has more than one loop section; a plurality of electrodes positioned along the ablation portion, the plurality of electrodes comprising a plurality of ablation electrodes; a first loop section comprising the innermost or distal most portion in the form of a round structure or of the three-dimensional spiral; optionally a second loop section, adjacent to the first loop section, wherein the second loop section does not include any ablation electrodes; a first ablation electrode positioned on the first loop section, the first ablation electrode having a first edge facing an interior of the round structure or the three dimensional spiral in a plan view; a second ablation electrode positioned on the first loop section, the second ablation electrode having a first edge facing an interior of the round structure or the three dimensional spiral in a plan view; a third ablation electrode positioned on the first loop section, the third ablation electrode having a first edge facing an interior of the round structure or the three dimensional spiral in a plan view; a fourth ablation electrode positioned on the first loop section, the fourth ablation electrode having a first edge facing an interior of the round structure or the three dimensional spiral in a plan view; wherein the first edge of the first ablation electrode and the first edge of the third ablation electrode substantially face each other in a pressed down position for the round structure or the compressed position, wherein the first edge of the second ablation electrode and the first edge of the fourth ablation electrode substantially face each other in the compressed position, wherein a distance across the interior of the three dimensional spiral in a plan view between the first edge of any two of the first ablation electrode, the second ablation electrode, the third ablation electrode, and the fourth ablation electrode, is not greater than 12mm in pressed down position for the round structure or the compressed position, and wherein the first edge of the first ablation electrode and the first edge of the third ablation electrode are substantially perpendicular to the first edge of the second ablation electrode and the first edge of the fourth ablation electrode in the pressed down or compressed position.
2. The ablation catheter of embodiment 1, wherein the material comprising the first loop section has a greater stiffness than the material comprising second loop section.
3. The ablation catheter of embodiment 1, wherein the first loop section is more proximal than the second loop section on the ablation portion.
4. The ablation catheter of embodiment 1, wherein the first ablation electrode produces a first ablation region, the second ablation electrode produces a second ablation region, the third ablation electrode produces a third ablation region, and the fourth ablation electrode produces a fourth ablation region.
5. The ablation catheter of embodiment 4, wherein the first ablation region, the second ablation region, the third ablation region, and the fourth ablation region overlap.
6. The ablation catheter of embodiment 5, wherein the overlapping ablation regions of the first ablation electrode, the second ablation electrode, the third ablation electrode, and the fourth ablation electrode form a single contiguous ablation region.
7. The ablation catheter of embodiment 1, wherein the first ablation electrode, the second ablation electrode, the third ablation electrode, and the fourth ablation electrode are evenly distributed relative to each other in the compressed position, such that each of the first ablation electrode, the second ablation electrode, the third ablation electrode, and the fourth ablation electrode occupies a corner or a side, in particular the center area of a side, of a rectangle in a plan view.
8. The ablation catheter of embodiment 7, wherein the rectangle is a square.
9. The ablation catheter of embodiment 1, further comprising a plurality of mapping electrodes on the second loop section.
10. An ablation catheter for treatment of patient tissue by delivery of high-voltage pulses comprising: a catheter shaft having an ablation portion at a distal end of the catheter shaft, wherein the ablation portion comprises at least one, optionally two loop sections forming a round structure or a three-dimensional spiral, wherein optionally the at least two loop sections configured to transition between an uncompressed position where the ablation portion is not restricted by any external force and a compressed position where the ablation portion is compressed by an external force when the catheter has more than one loop section; a plurality of electrodes positioned along the ablation portion, the plurality of electrodes comprising a plurality of ablation electrodes; a first loop section comprising the innermost or distal most portion in the form of a round structure or of the three-dimensional spiral; optionally a second loop section, adjacent to the first loop section, wherein the second loop section does not include any ablation electrodes; a first ablation electrode positioned on the first loop section, the first ablation electrode having a first edge facing an interior of the round structure or the three dimensional spiral in a plan view; a second ablation electrode positioned on the first loop section, the second ablation electrode having a first edge facing an interior of the round structure or the three dimensional spiral in a plan view; a third ablation electrode positioned on the first loop section, the third ablation electrode having a first edge facing an interior of the round structure or the three dimensional spiral in a plan view; a fourth ablation electrode positioned on the first loop section, the fourth ablation electrode having a first edge facing an interior of round structure or the three dimensional spiral in a plan view; a fifth ablation electrode positioned on the first loop section, the fifth ablation electrode having a first edge facing an interior of the round structure or the three dimensional spiral in a plan view; a sixth ablation electrode positioned on the first loop section, the sixth ablation electrode having a first edge facing an interior of the round structure or the three dimensional spiral in a plan view; wherein a distance across the interior of the round structure or the three dimensional spiral in a plan view between the first edge of any two of the first ablation electrode, the second ablation electrode, the third ablation electrode, the fourth ablation electrode, the fifth ablation electrode, and the sixth ablation electrode, is not greater than 12mm in pressed down position for the round structure or the compressed position, and wherein the first ablation electrode, the second ablation electrode, the third ablation electrode, the fourth ablation electrode, the fifth ablation electrode, and the sixth ablation electrode, are evenly distributed relative to each other in the pressed down position or the compressed position, such that each of the first ablation electrode, the second ablation electrode, the third ablation electrode, the fourth ablation electrode, the fifth ablation electrode, and the sixth ablation electrode occupies a corner or a side, in particular the center area of a side, of a hexagon in a plan view.
11. The ablation catheter of embodiment 10, wherein the material comprising the first loop section has a greater stiffness than the material comprising second loop section.
12. The ablation catheter of embodiment 10, wherein the first ablation electrode produces a first ablation region, the second ablation electrode produces a second ablation region, the third ablation electrode produces a third ablation region, the fourth ablation electrode produces a fourth ablation region, the fifth ablation electrode produces a fifth ablation region, and the sixth ablation electrode produces a sixth ablation region.
13. The ablation catheter of embodiment 12, wherein the first ablation region, the second ablation region, the third ablation region, the fourth ablation region, the fifth ablation region, and the sixth ablation region overlap.
14. The ablation catheter of embodiment 13, wherein the overlapping ablation regions of the first ablation electrode, the second ablation electrode, the third ablation electrode, the fourth ablation electrode, the fifth ablation electrode, and the sixth ablation electrode form a single contiguous ablation region.
15. The ablation catheter of embodiment 10, further comprising a plurality of mapping electrodes on the second loop section.
16. An ablation catheter for treatment of patient tissue by delivery of high-voltage pulses comprising: a catheter shaft having an ablation portion at a distal end of the catheter shaft, wherein the ablation portion comprises at least one, optionally two loop sections forming a round structure or a three-dimensional spiral, wherein optionally the at least two loop sections configured to transition between an uncompressed position where the ablation portion is not restricted by any external force and a compressed position where the ablation portion is compressed by an external force when the catheter has more than one loop section; a plurality of electrodes positioned along the ablation portion, the plurality of electrodes comprising a plurality of ablation electrodes; a first loop section comprising the innermost or distal most portion in the form of a round structure or of the three-dimensional spiral; optionally a second loop section, adjacent to the first loop section, wherein the second loop section does not include any ablation electrodes; a first ablation electrode positioned on the first loop section, the first ablation electrode having a first edge facing an interior of the round structure or the three dimensional spiral in a plan view; a second ablation electrode positioned on the first loop section, the second ablation electrode having a first edge facing an interior of the round structure or the three dimensional spiral in a plan view; wherein a distance across the interior of the round structure or the three dimensional spiral in a plan view between the first edge of the first ablation electrode and the first edge of the second ablation electrode, is not greater than 12mm in the pressed down position for the round structure or compressed position, and wherein the first ablation electrode produces a first ablation region and the second ablation electrode produces a second ablation region, and the first ablation region and the second ablation region overlap to form a single contiguous ablation region.
17. A method for treatment of patient tissue by delivery of high-voltage pulses comprising: providing an ablation catheter having an ablation portion including a plurality of ablation electrodes, the ablation portion comprising at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes, wherein optionally the at least two loop sections are configured to transition between an uncompressed position where the ablation portion is not restricted by any external force and a compressed position where the ablation portion is compressed by an external force when the catheter has more than one loop section, wherein a first loop section comprising the innermost or distal most portion in the form of a round structure or of the three-dimensional spiral includes a plurality of ablation electrodes, each of the plurality of ablation electrodes comprising a first edge facing an interior of the round structure or the three dimensional spiral in a plan view, wherein a distance across the interior of the round structure or the three dimensional spiral in a plan view between the first edge of any two of the ablation electrodes is not greater than 12mm in the pressed down position for the round structure or the compressed position; and energizing the plurality of ablation electrodes with high voltage charge-balanced pulsed electric fields preferably delivered in a monopolar arrangement to form a single contiguous ablation region.
18. The method of embodiment 17, wherein the high voltage charge-balanced pulsed electric fields are delivered in a combination of a monopolar arrangement and a bipolar arrangement.
19. The method of embodiment 18, a first electrode and a second electrode are energized with said pulsed electric fields in a bipolar arrangement.
20. The method of embodiment 17, wherein the high voltage charge balanced pulsed electric field energy is applied in the form of biphasic pulses comprising a positive and a negative pulse section, whereby the charge of the positive section balances out the charge of the negative section.
21. The method of embodiment 20, wherein energizing the plurality of electrodes with high voltage charge-balanced pulsed electric fields comprises a biphasic pulse followed by an inverse biphasic pulse separated by an interphase delay within a range of 1 µs to 100 µs.
22. The method of embodiment 20, wherein a pulse shape of the biphasic pulses is an exponential-decay.
23. The method of embodiment 20, wherein a voltage amplitude of pulses delivered to the ablation electrodes is greater than 1 kV.
24. The method of embodiment 20, wherein a voltage amplitude of pulses delivered to the ablation electrodes is greater than 2.5 kV.
25. The method of embodiment 20, wherein a voltage amplitude of pulses delivered to the ablation electrodes is between 2.5 kV and 10 kV.
26. The method of embodiment 20, wherein a pulse width of pulses delivered to the ablation electrodes is greater than 0.5 µs.
27. The method of embodiment 20, wherein a pulse width of pulses delivered to the ablation electrodes is between 0.5 µs and 30 µs.
28. The method of embodiment 17, wherein energizing the plurality of electrodes with high voltage charge-balanced pulsed electric fields is synchronized to a QRS cycle, P wave and/or T wave.
29. The method of embodiment 17, wherein a sterile irrigation fluid is applied at a treatment site, whereby distilled water or a physiological saline solution having a salinity of no more than 1%, is used as irrigation fluid.
30. A method for treatment of patient tissue by delivery of high-voltage pulses comprising: providing an ablation catheter having an ablation portion including a plurality of ablation electrodes, the ablation portion comprising at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes, optionally wherein the at least two loop sections are configured to transition between an uncompressed position where the ablation portion is not restricted by any external force and a compressed position where the ablation portion is compressed by an external force when the catheter has more than one loop section, wherein a first loop section comprising the innermost or distal most portion in the form of a round structure or of the three-dimensional spiral includes a plurality of ablation electrodes, each of the plurality of ablation electrodes comprising a first edge facing an interior of the round structure or the three dimensional spiral in a plan view, wherein a distance across the interior of the round structure or the three dimensional spiral in a plan view between the first edge of any two of the ablation electrodes is not greater than 12mm in a pressed down position for the round structure or the compressed position; and energizing the plurality of ablation electrodes with high voltage charge-balanced pulsed electric fields preferably delivered in a monopolar arrangement to form a single contiguous ablation region.
31. The method of embodiment 30, wherein a first electrode and a second electrode are energized with said pulsed electric fields in a bipolar arrangement.
32. The method of embodiment 30, wherein the high voltage charge balanced pulsed electric field energy is applied in the form of biphasic pulses comprising a positive and a negative pulse section, whereby the charge of the positive section balances out the charge of the negative section.
33. The method of embodiment 32, wherein energizing the plurality of electrodes with high voltage charge-balanced pulsed electric fields comprises a biphasic pulse followed by an inverse biphasic pulse separated by an interphase delay within a range of 1 µs to 100 µs.
34. The method of embodiment 32, wherein a pulse shape of the biphasic pulses is an exponential-decay.
35. The method of embodiment 32, wherein a voltage amplitude of pulses delivered to the ablation electrodes is greater than 1 kV.
36. The method of embodiment 32, wherein a voltage amplitude of pulses delivered to the ablation electrodes is greater than 2.5 kV.
37. The method of embodiment 32, wherein a voltage amplitude of pulses delivered to the ablation electrodes is between 2.5 kV and 10 kV.
38. The method of embodiment 32, wherein a pulse width of pulses delivered to the ablation electrodes is greater than 0.5 µs.
39. The method of embodiment 32, wherein a pulse width of pulses delivered to the ablation electrodes is between 0.5 µs and 30 µs.
40. The method of embodiment 30, wherein energizing the plurality of electrodes with high voltage charge-balanced pulsed electric fields is synchronized to a QRS cycle, P wave and/or T wave.
41. The method of embodiment 30, wherein a sterile irrigation fluid is applied at a treatment site, whereby distilled water or a physiological saline solution having a salinity of no more than 1%, is used as irrigation fluid.
42. An ablation catheter for treatment of patient tissue by delivery of high-voltage pulses comprising: a catheter shaft having an ablation portion at a distal end of the catheter shaft, wherein the ablation portion comprises at least one, optionally two loop sections forming a round structure or a three-dimensional spiral, wherein optionally the at least two loop sections configured to transition between an uncompressed position where the ablation portion is not restricted by any external force and a compressed position where the ablation portion is compressed by an external force when the catheter has more than one loop section; a plurality of ablation electrodes arranged along a first loop section comprising the innermost or distal most portion in the form of a round structure or of the three-dimensional spiral; optionally a second loop section, adjacent to the first loop section, wherein the second loop section does not include any ablation electrodes; wherein the plurality of ablation electrodes are arranged at vertices or a side, in particular the center area of a side, of a rectangular or hexagonal polygon in a plan view relative to each other in pressed down position for the round structure or the compressed position, and wherein the distance between any two ablation electrodes does not exceed 12 mm in pressed down position for the round structure or the compressed position.
43. The ablation catheter of embodiment 42, wherein the material comprising the first loop section has a greater stiffness than the material comprising second loop section.
44. The ablation catheter of embodiment 42, wherein the first loop section is more proximal than the second loop section on the ablation portion.
45. The ablation catheter of embodiment 42, further comprising at least four ablation electrodes, wherein a first ablation electrode produces a first ablation region, a second ablation electrode produces a second ablation region, a third ablation electrode produces a third ablation region, and a fourth ablation electrode produces a fourth ablation region.
46. The ablation catheter of embodiment 45, wherein the first ablation region, the second ablation region, the third ablation region, and the fourth ablation region overlap.
47. The ablation catheter of embodiment 46, wherein the overlapping ablation regions of the first ablation electrode, the second ablation electrode, the third ablation electrode, and the fourth ablation electrode form a single contiguous ablation region.
48. The ablation catheter of embodiment 42, wherein the polygon is a rectangle and the first ablation electrode, the second ablation electrode, the third ablation electrode, and the fourth ablation electrode are evenly distributed relative to each other in the compressed position, such that each of the first ablation electrode, the second ablation electrode, the third ablation electrode, and the fourth ablation electrode occupies a corner or a side, in particular the center area of a side, of the rectangle in the plan view.
49. The ablation catheter of embodiment 48, wherein the rectangle is a square.
50. The ablation catheter of embodiment 42, further comprising a plurality of mapping electrodes on the second loop section.
51. The ablation catheter of embodiment 42, further comprising at least six ablation electrodes, wherein a first ablation electrode produces a first ablation region, a second ablation electrode produces a second ablation region, a third ablation electrode produces a third ablation region, a fourth ablation electrode produces a fourth ablation region, a fifth ablation electrode produces a fifth ablation region, and a sixth ablation electrode produces a sixth ablation region.
52. The ablation catheter of embodiment 51, wherein the first ablation region, the second ablation region, the third ablation region, the fourth ablation region, the fifth ablation region, and the sixth ablation region overlap.
53. The ablation catheter of embodiment 52, wherein the overlapping ablation regions of the first ablation electrode, the second ablation electrode, the third ablation electrode, the fourth ablation electrode, the fifth ablation electrode, and the sixth ablation electrode form a single contiguous ablation region.
54. The ablation catheter of embodiment 53, further comprising a plurality of mapping electrodes on the second loop section.

## Claims

1. An ablation catheter for treatment of patient tissue by delivery of high-voltage pulses comprising:
a catheter shaft having an ablation portion at a distal end of the catheter shaft, wherein the ablation portion comprises at least one, optionally two loop sections forming a round structure or a three-dimensional spiral, wherein optionally the at least two loop sections configured to transition between an uncompressed position where the ablation portion is not restricted by any external force and a compressed position where the ablation portion is compressed by an external force when the catheter has more than one loop section;
a plurality of electrodes positioned along the ablation portion, the plurality of electrodes comprising a plurality of ablation electrodes;
a first loop section comprising the innermost or distal most portion in the form of a round structure or of the three-dimensional spiral;
optionally a second loop section, adjacent to the first loop section, wherein the second loop section does not include any ablation electrodes;
a first ablation electrode positioned on the first loop section, the first ablation electrode having a first edge facing an interior of the round structure or the three dimensional spiral in a plan view;
a second ablation electrode positioned on the first loop section, the second ablation electrode having a first edge facing an interior of the round structure or the three dimensional spiral in a plan view;
a third ablation electrode positioned on the first loop section, the third ablation electrode having a first edge facing an interior of the round structure or the three dimensional spiral in a plan view;
a fourth ablation electrode positioned on the first loop section, the fourth ablation electrode having a first edge facing an interior of the round structure or the three dimensional spiral in a plan view;
optionally a fifth ablation electrode positioned on the first loop section, the fifth ablation electrode having a first edge facing an interior of the round structure or the three dimensional spiral in a plan view;
optionally a sixth ablation electrode positioned on the first loop section, the sixth ablation electrode having a first edge facing an interior of the round structure or the three dimensional spiral in a plan view;
wherein a distance across the interior of the round structure or the three dimensional spiral in a plan view between the first edge of any two of the first ablation electrode, the second ablation electrode, the third ablation electrode, the fourth ablation electrode, the optional fifth ablation electrode, and the optional sixth ablation electrode, is not greater than 12mm in pressed down position for the round structure or the compressed position, and optionally
wherein the first edge of the first ablation electrode and the first edge of the third ablation electrode are substantially perpendicular to the first edge of the second ablation electrode and the first edge of the fourth ablation electrode in the pressed down or compressed position, or wherein the first ablation electrode, the second ablation electrode, the third ablation electrode, the fourth ablation electrode, the optional fifth ablation electrode, and the optional sixth ablation electrode, are evenly distributed relative to each other in the pressed down or compressed position, such that each of the first ablation electrode, the second ablation electrode, the third ablation electrode, the fourth ablation electrode, the optional fifth ablation electrode, and the optional sixth ablation electrode occupies a corner or a side, in particular the center area of a side, of a square or optional a hexagon in a plan view.

2. The ablation catheter of claim 1, wherein the material comprising the first loop section has a greater stiffness than the material comprising second loop section.

3. The ablation catheter of claim 1 or 2, wherein the ablation portion comprises only one loop section.

4. The ablation catheter of any of the preceding claims, wherein the first ablation electrode produces a first ablation region, the second ablation electrode produces a second ablation region, the third ablation electrode produces a third ablation region, and the fourth ablation electrode produces a fourth ablation region.

5. The ablation catheter of claim 4, wherein the first ablation region, the second ablation region, the third ablation region, and the fourth ablation region overlap.

6. The ablation catheter of claim 5, wherein the overlapping ablation regions of the first ablation electrode, the second ablation electrode, the third ablation electrode, and the fourth ablation electrode form a single contiguous ablation region.

7. The ablation catheter of any of the preceding claims, wherein the first ablation electrode, the second ablation electrode, the third ablation electrode, and the fourth ablation electrode are evenly distributed relative to each other in the pressed down or compressed position, such that each of the first ablation electrode, the second ablation electrode, the third ablation electrode, and the fourth ablation electrode occupies a corner or a side, in particular the center area of a side, of a rectangle in a plan view.

8. The ablation catheter of claim 7, wherein the rectangle is a square.

9. The ablation catheter any of the preceding claims, further comprising a plurality of mapping electrodes on the second loop section.

10. An ablation catheter for treatment of patient tissue by delivery of high-voltage pulses comprising: a catheter shaft having an ablation portion at a distal end of the catheter shaft, wherein the ablation portion comprises one loop section; a plurality of electrodes positioned along the ablation portion, the plurality of electrodes comprising a plurality of ablation electrodes; the loop section comprising the distal most portion in the form of a round structure; a first ablation electrode positioned on the loop section; a second ablation electrode positioned on the loop section; a third ablation electrode positioned on the loop section; a fourth ablation electrode positioned on the loop section; an optional fifth ablation electrode positioned on the loop section; an optional sixth ablation electrode positioned on the loop section; wherein a distance across the interior of the round structure in a plan view between interior aspects of any two electrodes, is not greater than 12mm in a deployed position.

11. The ablation catheter of claim 10, wherein the ablation catheter has four ablation electrodes and the round structure has the shape of a circular structure being circular, oval, elliptic or curved enclosing more than 180 degrees.

12. The ablation catheter of claim 10, wherein the first ablation electrode produces a first ablation region, the second ablation electrode produces a second ablation region, the third ablation electrode produces a third ablation region, the fourth ablation electrode produces a fourth ablation region, the fifth ablation electrode produces a fifth ablation region, and the sixth ablation electrode produces a sixth ablation region.

13. The ablation catheter of claim 12, wherein the first ablation region, the second ablation region, the third ablation region, the fourth ablation region, the fifth ablation region, and the sixth ablation region overlap.

14. The ablation catheter of claim 13, wherein the overlapping ablation regions of the first ablation electrode, the second ablation electrode, the third ablation electrode, the fourth ablation electrode, the fifth ablation electrode, and the sixth ablation electrode form a single contiguous ablation region.

15. A method for treatment of patient tissue by delivery of high-voltage pulses including: providing an ablation catheter having an ablation portion including a plurality of ablation electrodes, the ablation portion including at least one, optionally two loop sections forming a round structure or a three dimensional spiral at a distal end and a proximal end adapted to connect to an electronic control unit configured to provide monopolar, bipolar, or a combination of monopolar and bipolar energy a plurality of ablation electrodes, wherein when the catheter has at least two loop sections, they are configured to transition between an uncompressed position where the ablation portion is not restricted by any external force and a compressed position where the ablation portion is compressed by an external force, wherein the catheter has a first loop section, wherein the first loop section includes the innermost portion of the round structure or the three-dimensional spiral and further includes a plurality of ablation electrodes, each of the plurality of ablation electrodes including a first edge facing an interior of the round structure or the three dimensional spiral in a plan view, wherein a distance across the interior of the round structure or the three dimensional spiral in a plan view between the first edge of any two of the ablation electrodes is not greater than 12mm in a pressed down position or the compressed position; and energizing the plurality of ablation electrodes with high voltage charge-balanced pulsed electric fields, preferably in a monopolar arrangement to form a single contiguous ablation region, wherein the electrodes of the plurality of ablation electrodes have a first configuration, wherein the same number of electrodes have a "+" polarity and a "-" polarity, or the number of electrodes with one polarity differs from the number of electrodes with the other polarity by only one (1).
